# EUROPEAN PATENT SPECIFICATION

(11) **EP 4 153 994 B1**
(45) Date of publication and mention of the grant of the patent: **12.11.2025**
(21) Application number: 21809426.6
(22) Date of filing: 19.05.2021
(51) Int. Cl.: G01N 33/50, G01N 33/542, C12Q 1/02

(54) **USE OF AUTOMATED PLATFORMS FOR PREPARATION OF BIOMARKER AND ROMANOWSKY-TYPE STAINED SAMPLE PRINTED ON A SLIDE**
VERWENDUNG VON AUTOMATISIERTEN PLATTFORMEN ZUR HERSTELLUNG VON AUF EINEM OBJEKTTRÄGER GEDRUCKTEN BIOMARKER- UND ROMANOWSKY-GEFÄRBTEN PROBEN
UTILISATION DE PLATEFORMES AUTOMATISÉES POUR LA PRÉPARATION D'UN BIOMARQUEUR ET D'UN ÉCHANTILLON À COLORATION DE TYPE ROMANOWSKY IMPRIMÉ SUR UNE LAME

(30) Priority: 20.05.2020 US 202063027720 P
(43) Date of publication of application: 29.03.2023
(73) Proprietor: Roche Diagnostics Hematology, Inc., Indianapolis IN 46256 (US); F. Hoffmann-La Roche AG, 4070 Basel (CH); Roche Diagnostics GmbH, 68305 Mannheim (DE)
(72) Inventor: NEUBAUER-PERCHUC, Anna Maria, 4070 Basel (CH); HOOGENDIJK, Jan-Gerrit, 4070 Basel (CH); GRETHER, Nicole, 4070 Basel (CH); ZAHNISER, David J., Indianapolis, IN 46256 (US); MUI, Katherine K., Indianapolis, IN 46256 (US); TACKE, Michael, 68305 Muenchen (DE); TORRES-NAGEL, Nora, 68305 Muenchen (DE)
(74) Representative: Curcio, Mario
(86) International application number: PCT/US2021/033246
(87) International publication number: WO 2021/236829

(56) References cited:
- US-A- 6 007 996
- US-A1- 2002 177 149
- US-A1- 2006 160 169
- US-A1- 2018 007 319
- STEFANOVIC D ET AL: "Romanowsky-Giemsa as a counterstain for immunohistochemistry: optimizing a traditional reagent", BIOTECHNIC AND HISTOCHEMISTRY., vol. 88, no. 6, 8 May 2013 (2013-05-08), US, pages 329 - 335, XP093145113, ISSN: 1052-0295, DOI: 10.3109/10520295.2013.785595
- GROSSET ANDR�E-ANNE ET AL: "Hematoxylin and Eosin Counterstaining Protocol for Immunohistochemistry Interpretation and Diagnosis", vol. 27, no. 7, 1 August 2019 (2019-08-01), US, pages 558 - 563, XP093145127, ISSN: 1541-2016, Retrieved from the Internet <URL:https://dx.doi.org/10.1097/PAI.0000000000000626> DOI: 10.1097/PAI.0000000000000626
- PRYZWANSKY KATHERINE B. ET AL: "Immunocytochemical Distinction Between Primary and Secondary Granule Formation in Developing Human Neutrophils: Correlations With Romanowsky Stains", vol. 52, no. 2, 1 February 1979 (1979-02-01), pages 179 - 185, XP093145466, Retrieved from the Internet <URL:https://pdf.sciencedirectassets.com/778769/1-s2.0-S0006497120X71448/1-s2.0-S0006497120706944/main.pdf?X-Amz-Security-Token=IQoJb3JpZ2luX2VjEHgaCXVzLWVhc3QtMSJHMEUCIQD7+Q5ZCKHhGf7HpHbahYhZ0koBGaTy4nZefjHwwIH+MAIgP6A7gFDwR7eHNoeVuSLpyj18jn9YJTOB7M7WjcX6B8UqvAUIkP//////////ARAFGgwwNTkwMDM1NDY4NjUiDBI7y>
- MLAHIANI AMAL ET AL: "Enabling Histopathological Annotations on Immunofluorescent Images through Virtualization of Hematoxylin and Eosin", vol. 9, no. 1, 1 January 2018 (2018-01-01), IN, pages 1, XP093145404, ISSN: 2153-3539, Retrieved from the Internet <URL:https://pdf.sciencedirectassets.com/781441/1-s2.0-S2153353922X60069/1-s2.0-S2153353922003145/main.pdf?X-Amz-Security-Token=IQoJb3JpZ2luX2VjEHcaCXVzLWVhc3QtMSJIMEYCIQC2ycnufhfdKMpvpiCbGFikF6Q/C0i+qnF/bv3TuB0F/QIhAOUjyENDmbCuLD6BqPzRVXUkZH27eAgofT5XYoIcebUjKrwFCJD//////////wEQBRoMMDU5MDAzNTQ2ODY1Igzk2> DOI: 10.4103/jpi.jpi_61_17
- ROSE E. RASKIN ET AL: "Optimized immunocytochemistry using leukocyte and tissue markers on Romanowsky-stained slides from dogs and cats", VETERINARY CLINICAL PATHOLOGY, vol. 48, no. S1, 25 July 2019 (2019-07-25), United States, pages 88 - 97, XP055656915, ISSN: 0275-6382, DOI: 10.1111/vcp.12759

## Description

### BACKGROUND OF THE DISCLOSURE

The present disclosure relates generally to methods and systems for detecting, characterizing and morphological analysis of biomarker expression in cell samples. The methods allow for the use of automated platforms to stain cells for molecular biomarkers and Romanowsky-type staining for cell morphology. Cells that are prepared according to the disclosed methods can also be used in the diagnosis of certain conditions.

Cellular samples are useful for diagnostics including screening for and diagnosis using blood samples. For diagnosis using blood samples, typically one portion of the blood sample is used for morphological analysis. Samples stained for morphological analysis are generally not reusable. A separate portion of the blood sample is evaluated by flow cytometry to detect molecular markers. In cases where a portion of the sample is used for morphological analysis and a separate portion is used for flow cytometry, a one to one comparison between morphologically abnormal cells and the ones stained for molecular biomarkers is not possible. This workflow is also expensive and time consuming.

Stefanovic et al. disclose a protocol for using Romanowsky-Giemsa counterstaining on formalin fixed, paraffin embedded tissue sections that are stained immunohistochemically after antigen retrieval using hot acidic citrate buffer (Stefanovic et al., Romanowsky-Giemsa as a counterstain for immunohistochemistry: optimizing a traditional reagent, Biotechnic & Histochemistry 2013, 88:6, 329-335. https://doi.org/10.3109/10520295.2013.785595).

Grosset et al. disclose a protocol for using hematoxylin and eosin (H&E) as counterstain on formalin fixed, paraffin embedded tissue sections that are stained immunohistochemically (Grosset et al., Hematoxylin and eosin counterstaining protocol for immunohistochemistry interpretation and diagnosis, Appl Immunohistochem MolMorphol 2019, 27:558-563).

Pryzwansky et al. propose a method to permit immunofluorescent demonstration of primary and secondary granule markers in cells stained with Romanowsky agents (Pryzwansky et al., Immunocytochemical distinction between primary and secondary granule formation in developing human neutrophils: correlations with Romanowsky stains, Blood, Volume 53, Issue 2, 1979, Pages 179-185).

Lahiani et al. disclose an algorithm designed for the conversion of fluorescence images to brightfield hematoxylin and eosin (H&E) images (Lahiani et al., Enabling histopathological annotations on immunofluorescent images through virtualization of hematoxylin and eosin, J Pathol Inform 2018;9:1*).*

Raskin et al. disclose a protocol for immunocytochemically staining Romanowsky-stained specimens using antibodies against CD3ε, CD20, cytokeratin, lysozyme, Melan-A, MHCII, MUM1, Pax5, and vimentin (Raskin et al., Optimized immunocytochemistry using leukocyte and tissue markers on Romanowsky-stained slides from dogs and cats, Vet Clin Pathol. 2019; 48(Suppl. 1):88-97).

Accordingly, there exists a need for compositions and methods for sample preparation and analysis that allow for a comparison between morphologically abnormal cells and cells stained for molecular biomarkers.

### BRIEF DESCRIPTION OF THE DISCLOSURE

The present disclosure is generally related to a method for detecting a biomarker and morphology in a cell sample, the method comprising: contacting a cell sample with one or more biomarker-specific reagents that specifically binds to a biomarker in the cell sample, wherein the one or more biomarker-specific reagents comprises a fluorescent label; depositing the biomarker-stained cell sample on a solid support; analyzing the biomarker-stained cell sample for one or more biomarker; staining the biomarker-stained cell sample with a Romanowsky-type stain to obtain a Romanowsky-type stained cell sample; and analyzing the Romanowsky-type stained cell sample to determine morphology of at least one cell in the Romanowsky-type stained cell sample.

### BRIEF DESCRIPTION OF THE DRAWINGS

The disclosure will be better understood, and features, aspects and advantages other than those set forth above will become apparent when consideration is given to the following detailed description thereof. Such detailed description makes reference to the following drawings, wherein:
FIG. 1 shows fluorescent images of CD 45 staining and Romanowsky-type staining in a body fluid sample.
FIG. 2 shows fluorescent images of CD 45 and CD 20 staining using a multiplex staining approach. The boxed area shows CD 45 positive/CD 20 negative staining of the same cell.

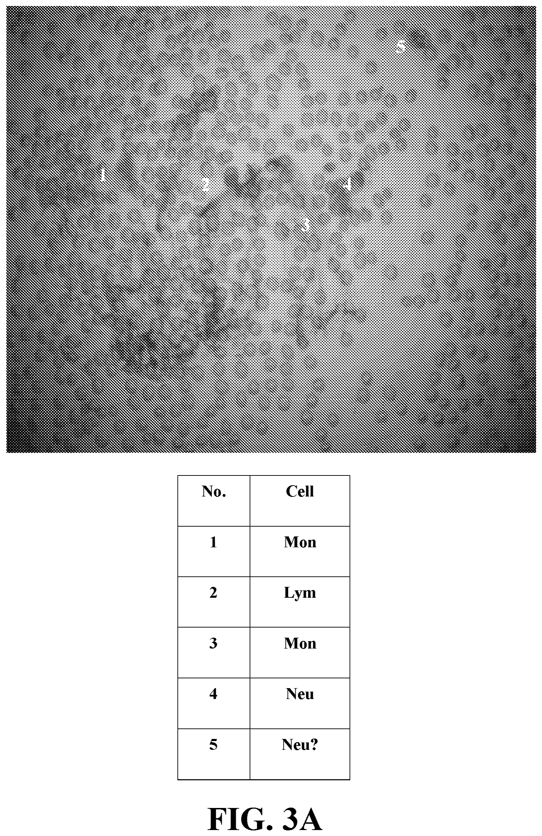

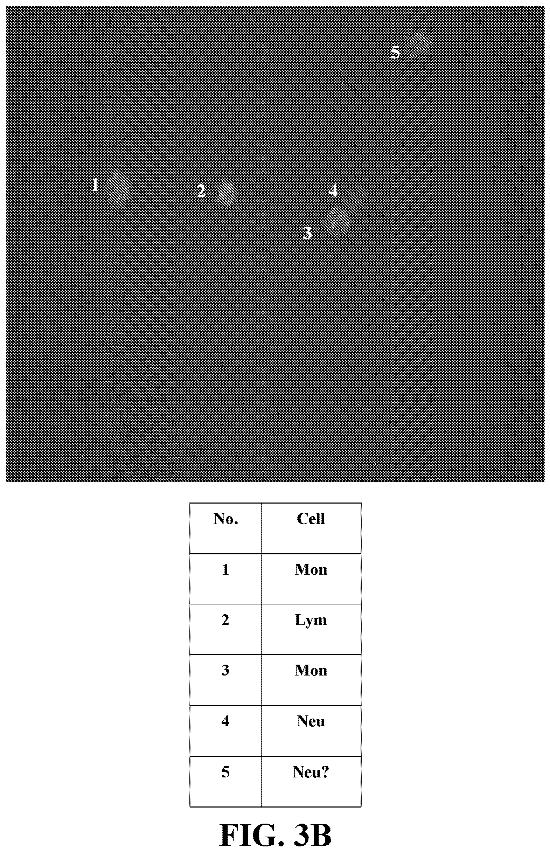

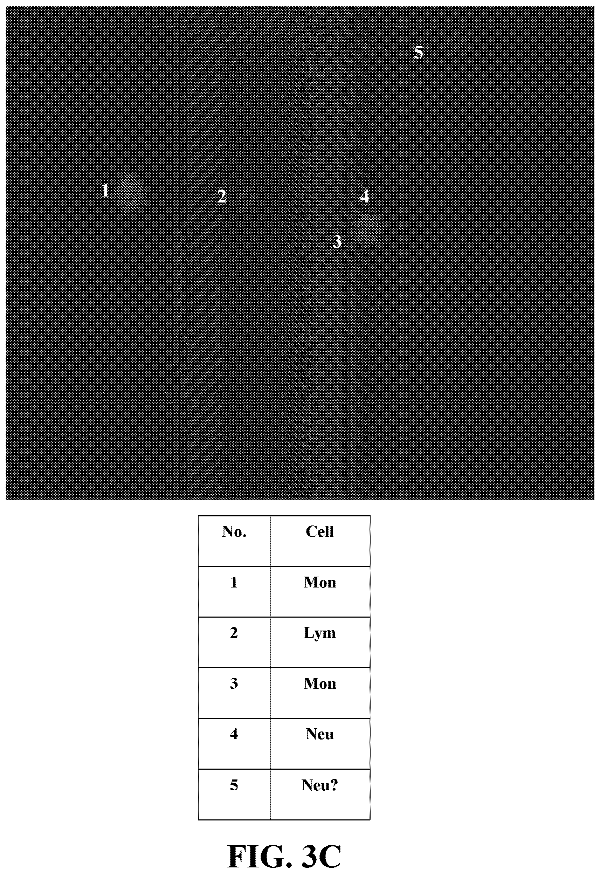

FIGS. 3A-3C show a cell sample stained with Romanowsky stain (FIG. 3A), CD 45 biomarker (FIG. 3B), and CD 14 APC (FIG. 3C). Mon = monocyte; Lym = lymphocyte; Neu = neutrophil; Neu? = possible neutrophil.

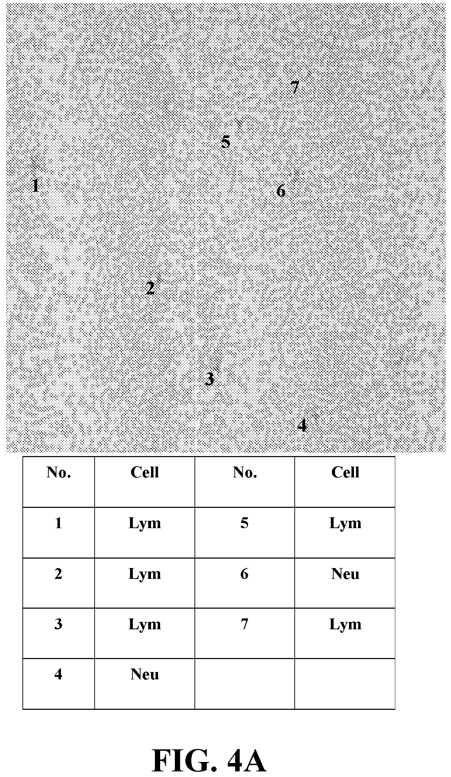

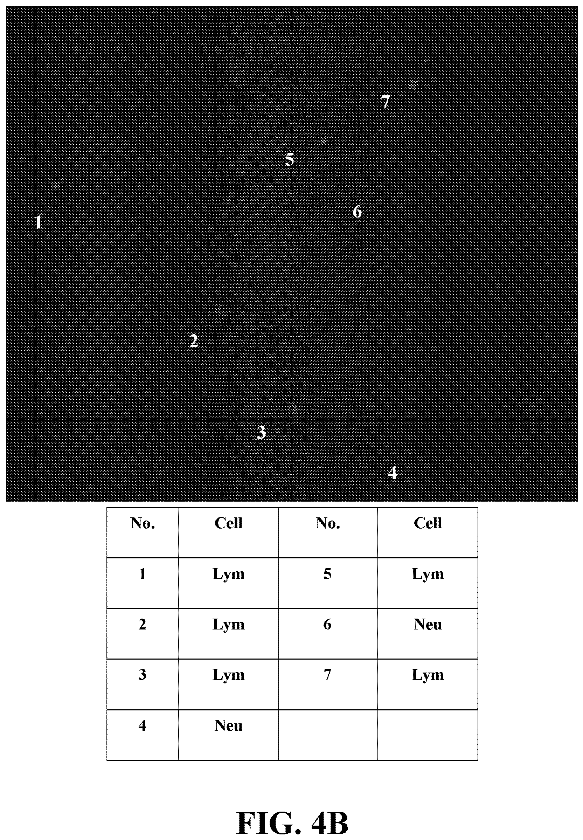

FIGS. 4A and 4B show a cell sample stained with Romanowsky stain (FIG. 4A) and CD 45 biomarker (FIG. 4B).

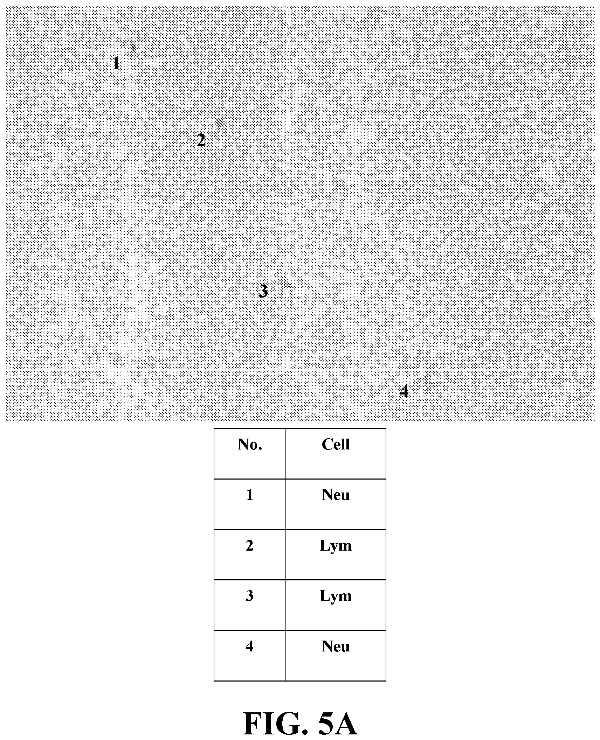

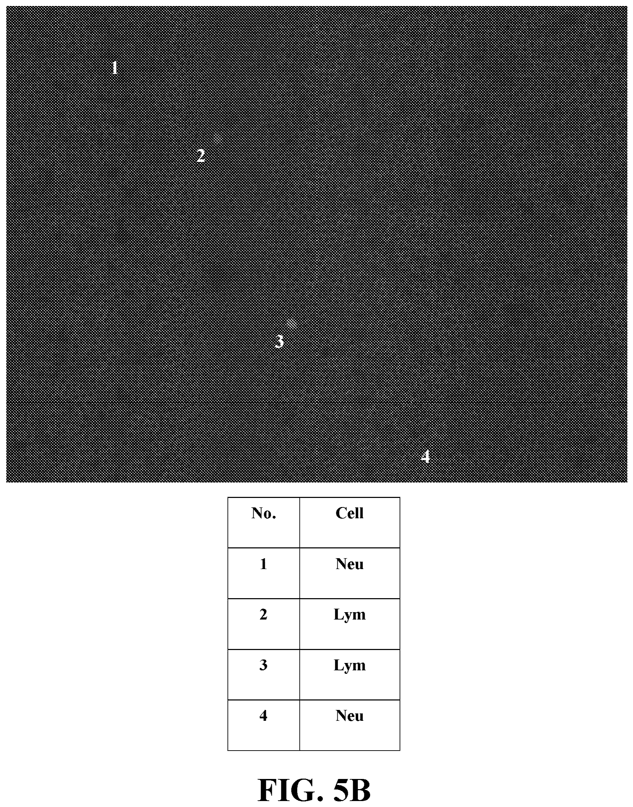

FIGS. 5A and 5B show a cell sample stained with Romanowsky stain (FIG. 5A) and CD 45 biomarker (FIG. 5B).

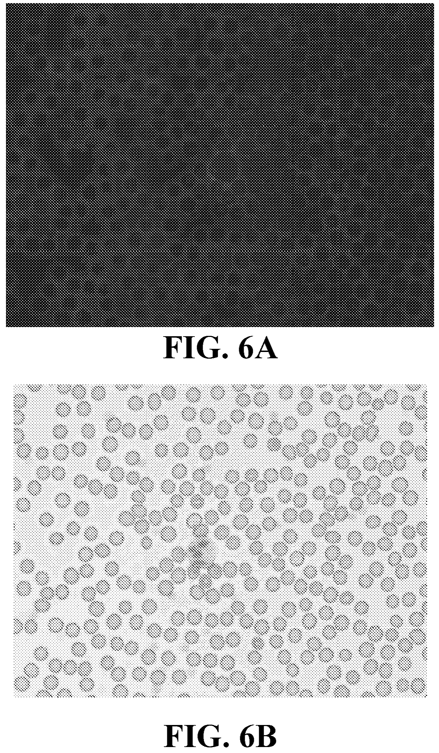

FIG. 6A and 6B show a cell sample stained for CD 45 (FIG. 6A) and Romanowsky stain (FIG. 6B).

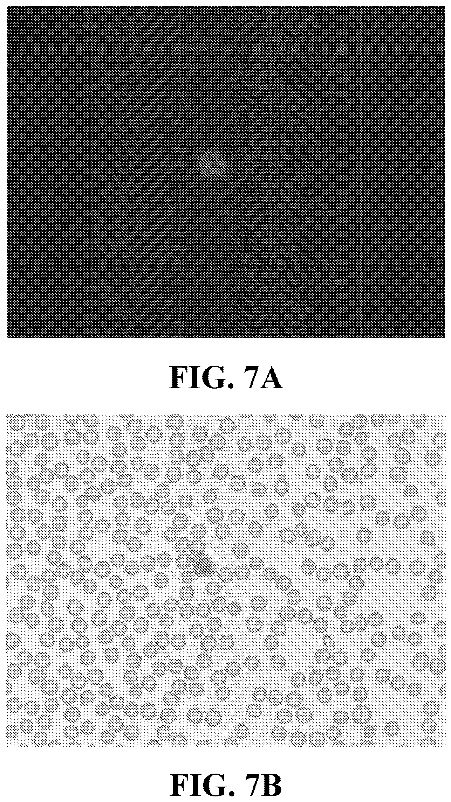

FIG. 7A and 7B show a cell sample stained for CD 45 (FIG. 7A) and Romanowsky stain (FIG. 7B).

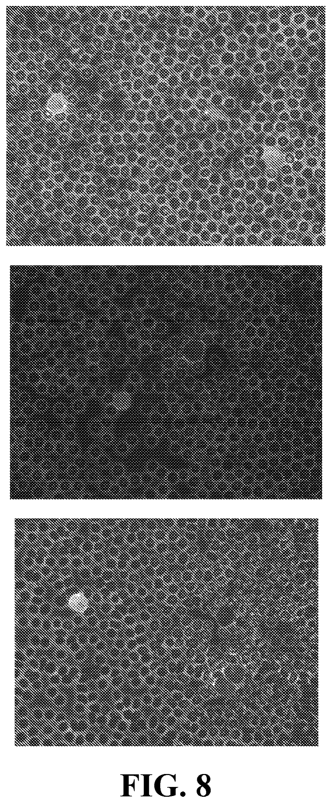

FIG. 8 shows a cell sample stained for CD 45.

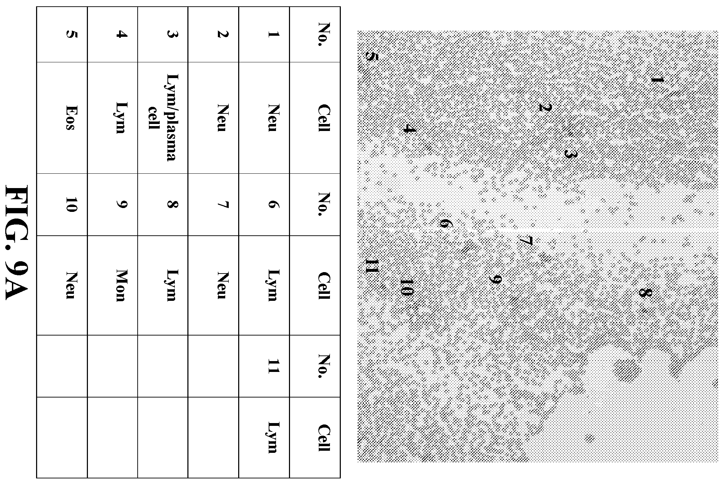

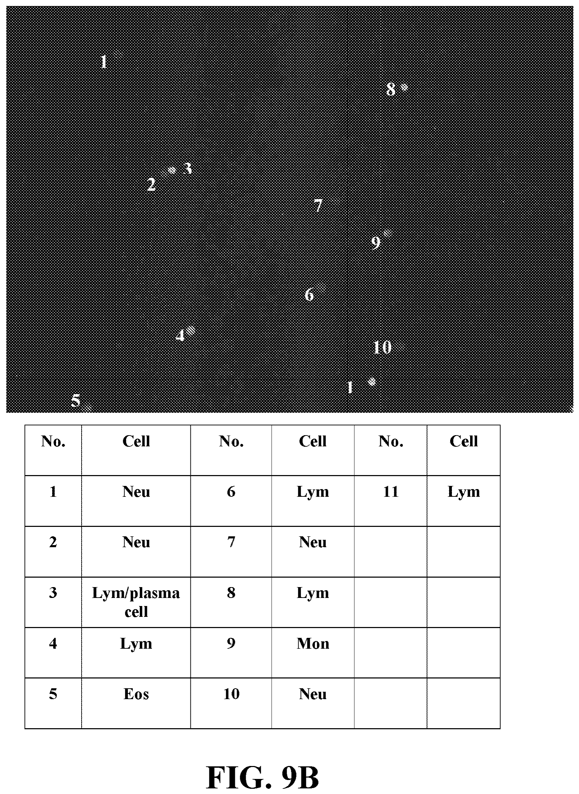

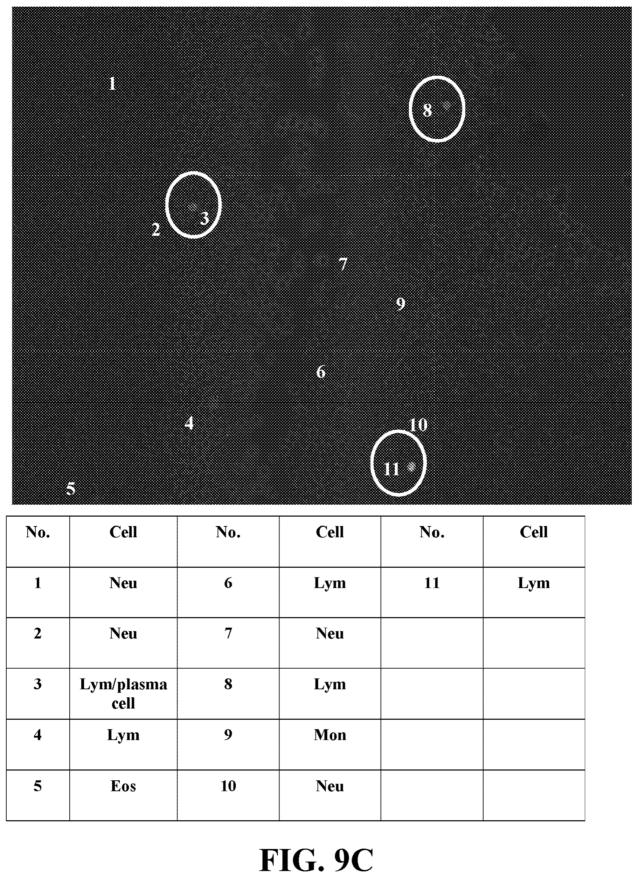

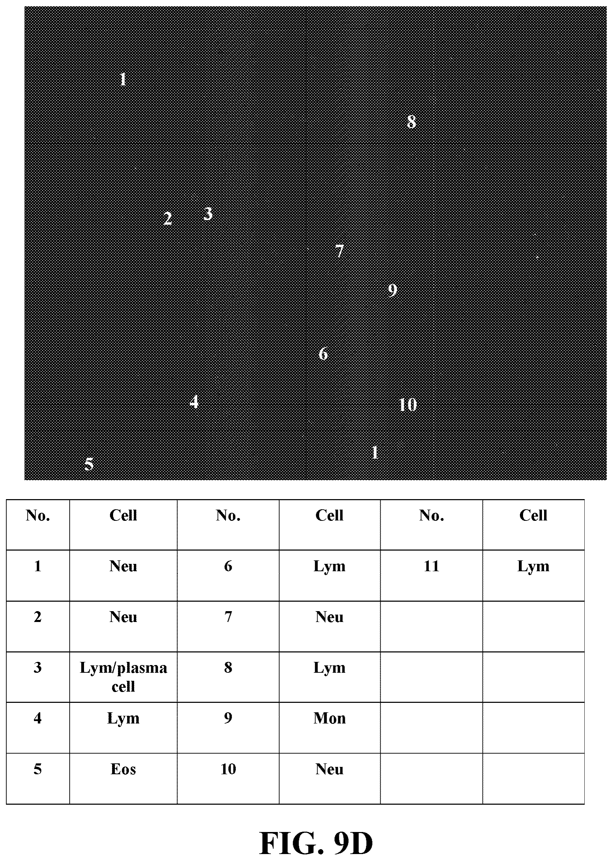

FIGS. 9A-9D show a cell sample Romanowsky stained (FIG. 9A), CD 45 stained (FIG. 9B), CD3 stained (FIG. 9C) with lymphocytes circled, and C19 stained (FIG. 9D).

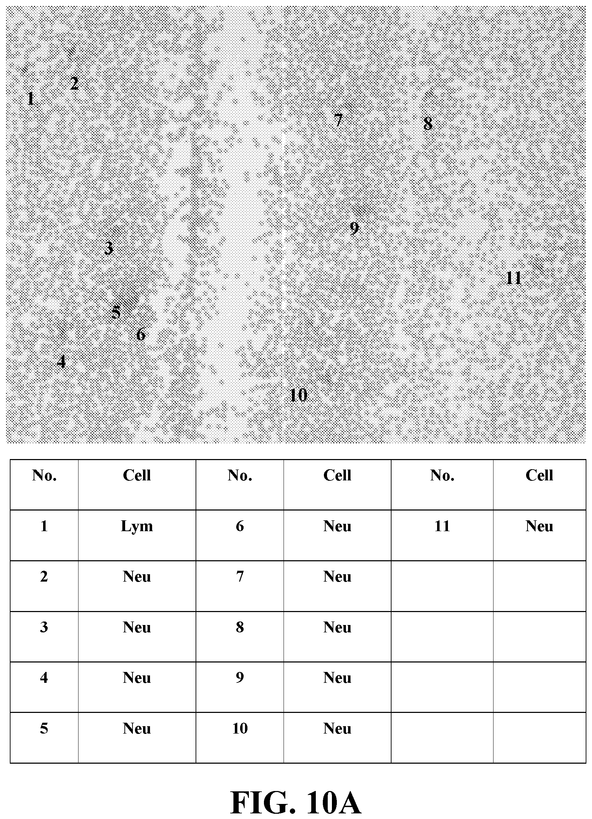

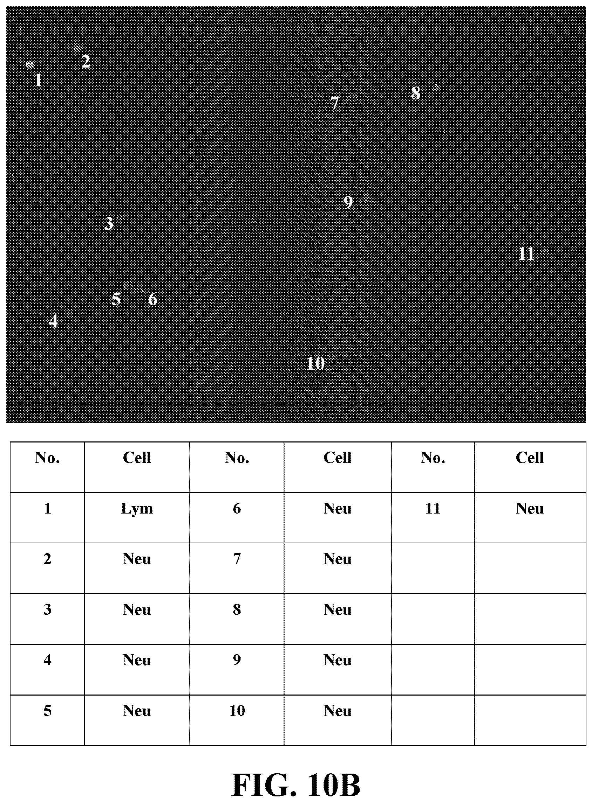

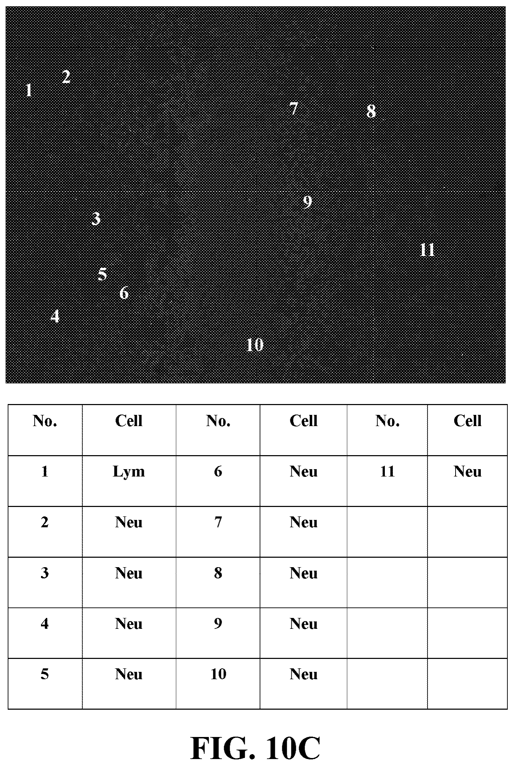

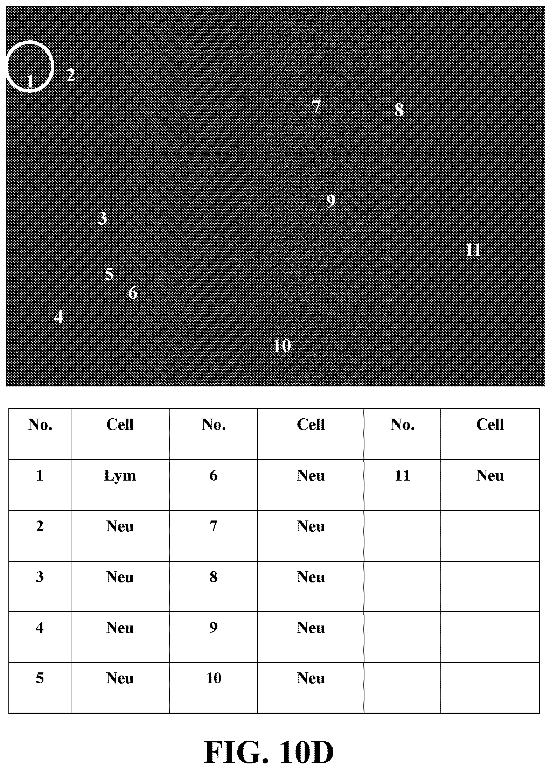

FIGS. 10A-10D show a cell sample Romanowsky stained (FIG. 10A), CD 45 stained (FIG. 10B), CD3 stained (FIG. 10C), and C19 stained (FIG. 10D) with lymphocyte circled.

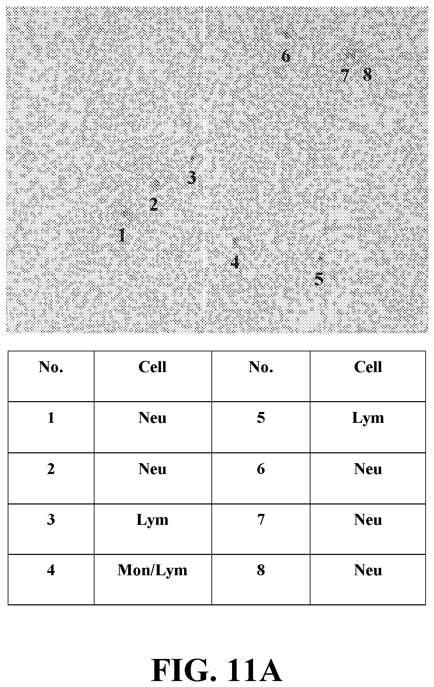

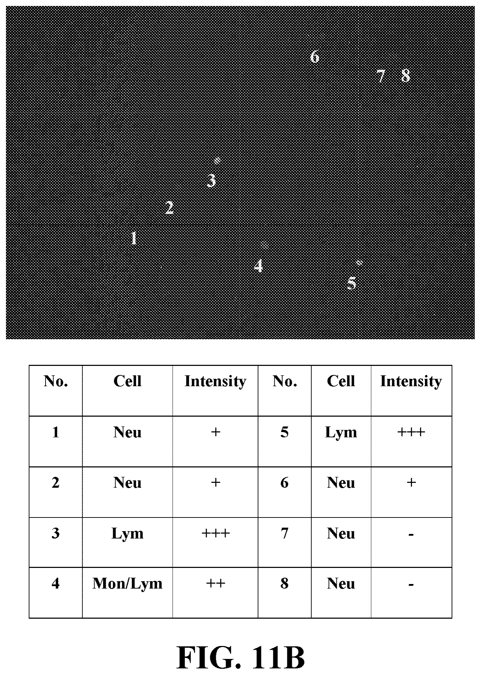

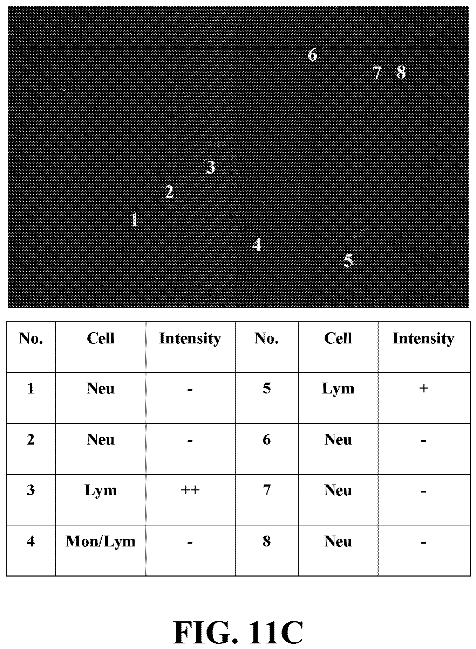

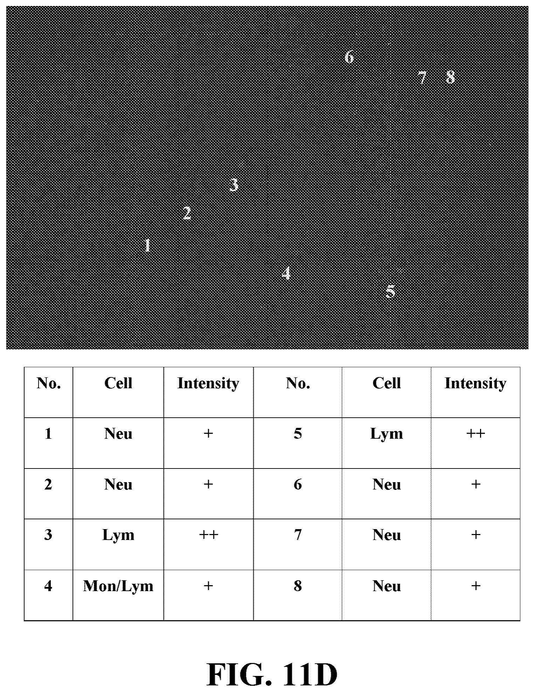

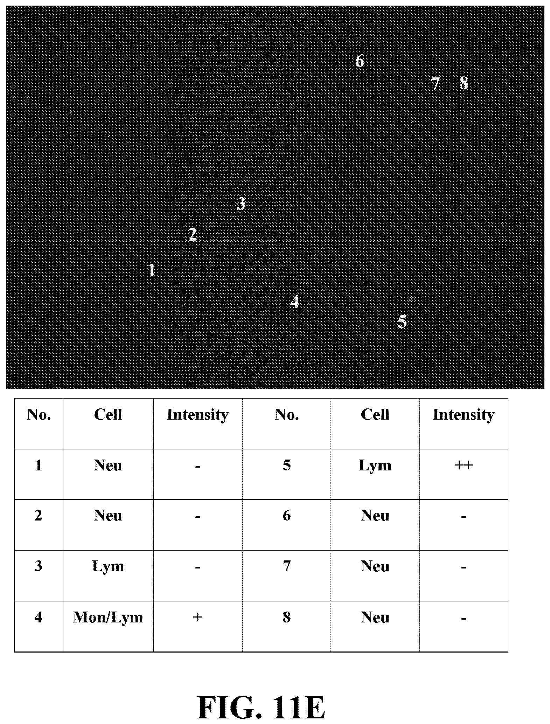

FIGS. 11A-11D show a cell sample Romanowsky stained (FIG. 11A), CD 45 stained (FIG. 11B), C19 stained (FIG. 11C), CD3 stained (FIG. 11D), and CD 16 and 56 stained (FIG. 11E).

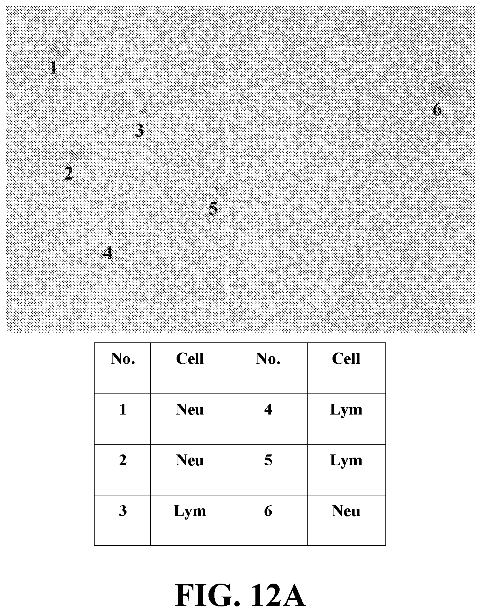

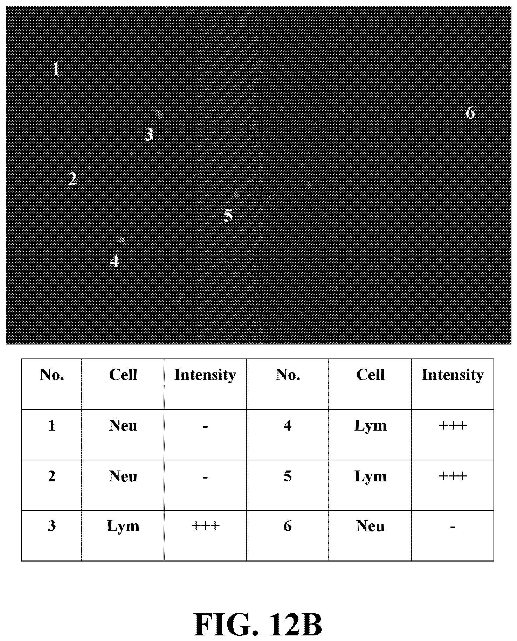

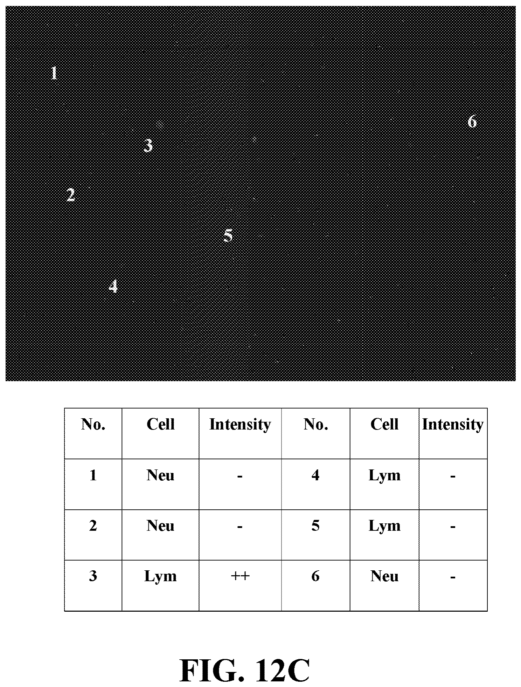

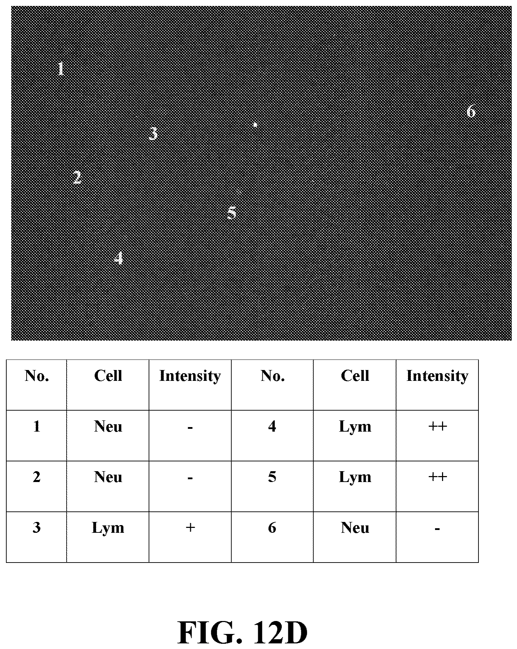

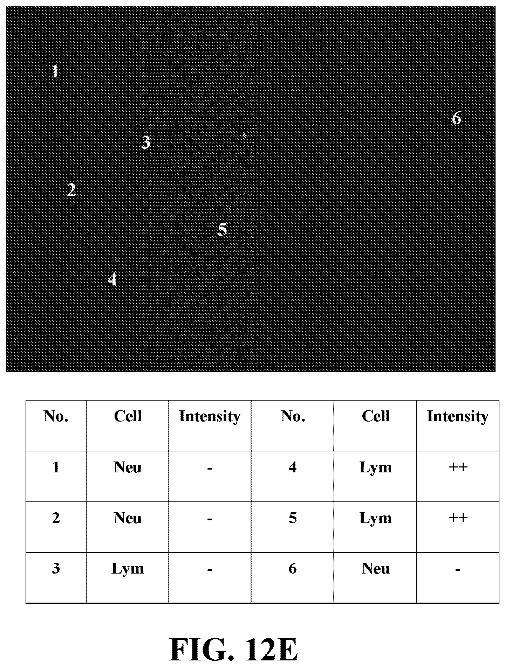

FIGS. 12A-12E show a cell sample Romanowsky stained (FIG. 12A), CD 45 stained (FIG. 12B), C19 stained (FIG. 12C), CD3 stained (FIG. 12D), and CD 16 and 56 stained (FIG. 12E).

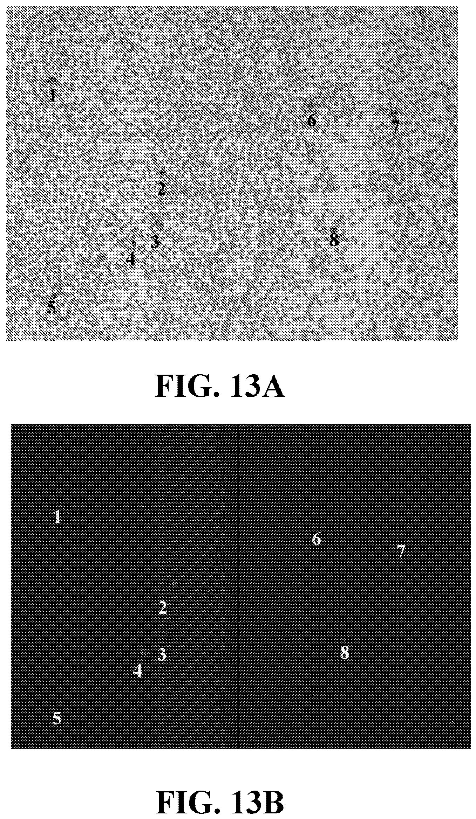

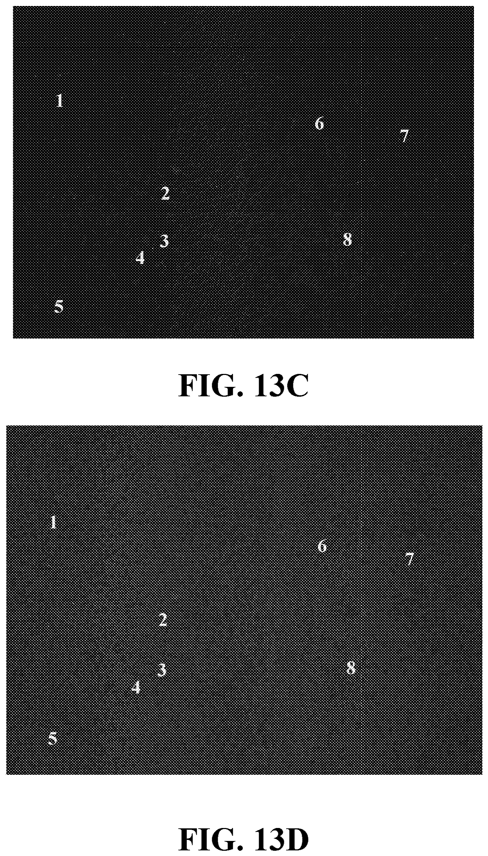

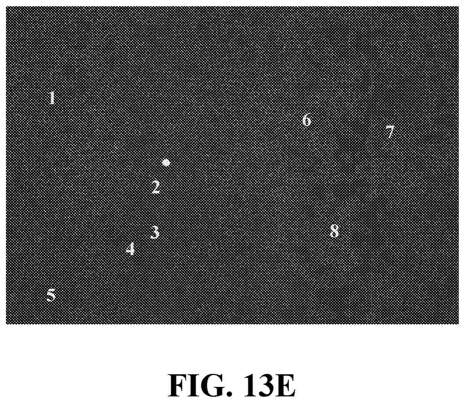

FIGS. 13A-13E show a cell sample Romanowsky stained (FIG. 13A), CD 45 stained (FIG. 13B), C19 stained (FIG. 13C), CD3 stained (FIG. 13D), and CD 16 and 56 stained (FIG. 13E).

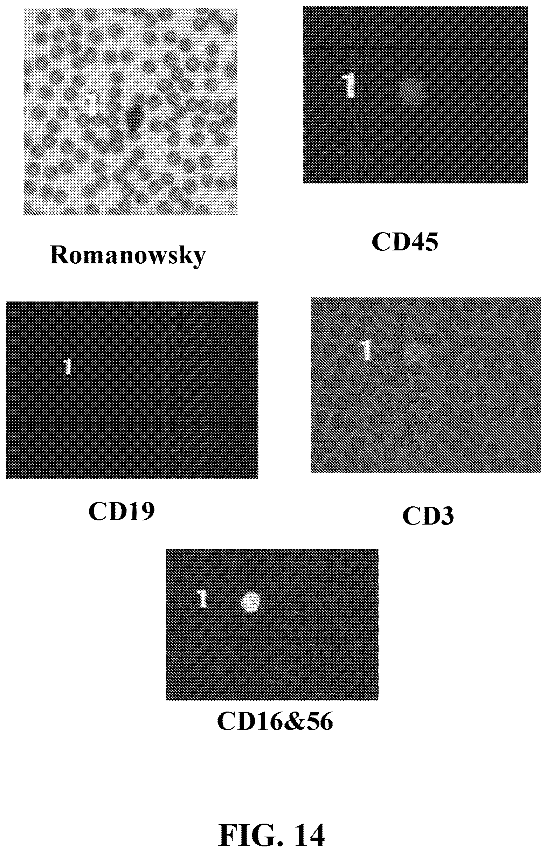

FIG. 14 shows the same cell sample Romanowsky stained, CD 45 stained, CD 19 stained, CD3 stained and CD 16 & 56 stained.
FIGS. 15A and 15B show a cell sample stained for CD3, CD4, CD8, CD16 and CD19 (FIG. 15A) and Romanowsky stained (FIG. 15B).
FIG. 16 shows a cell sample CD4 (BV750) stained, CD3 (AF488) stained, CD19 (AF594) stained, CD16 (AF647) stained, CD8 (JF549) stained, and a combined fluorescent image.
FIGS. 17A and 17B show a cell sample stained for CD3, CD4, CD8, CD16, and CD19 (FIG. 17A) and Romanowsky stained (FIG. 17B).
FIG. 18 shows individual panels of a cell sample stained for CD3 (AF488), CD4 (BV750), CD8 (JF549), CD16 (AF647), CD19 (AF594), and a combined fluorescent image.
FIGS. 19A and 19B show a cell sample stained for CD3, CD4, CD8, and CD16 (FIG. 19A) and Romanowsky stained (FIG. 19B).
FIG. 20 shows individual panels of a cell sample stained for CD3 (AF488), CD4 (BV750), CD8 (JF549), CD16 (AF647), CD19 (AF594), and a combined fluorescent image.
FIGS. 21A and 21B show a cell sample stained for CD3, CD4, CD8, and CD16 (FIG. 21A) and Romanowsky stained (FIG. 21B).
FIG. 22 shows individual panels of a cell sample stained for CD3 (AF488), CD4 (BV750), CD8 (JF549), CD16 (AF647), CD19 (AF594), and a combined fluorescent image.

### DETAILED DESCRIPTION

Unless defined otherwise, all technical and scientific terms used herein have the same meaning as commonly understood by one of ordinary skill in the art to which the disclosure belongs. Although any methods and materials similar to or equivalent to those described herein can be used in the practice or testing of the present disclosure, the preferred methods and materials are described below.

When introducing elements of the present disclosure or the various versions, embodiment(s) or aspects thereof, the articles "a", "an", "the" and "said" are intended to mean that there are one or more of the elements. The terms "comprising", "comprise," "comprises," "including" and "having" are intended to be inclusive and mean that there may be additional elements other than the listed elements.

As used herein, the term "subject" or "individual" is a mammal. Suitable mammals include, for example, domesticated animals (e.g., cows, sheep, cats, dogs, and horses), primates (e.g., humans and non-human primates such as monkeys), rabbits, and rodents (e.g., mice and rats).

As used herein, "sample" refers to any material obtained from a subject capable of being tested for the presence or absence of a biomarker. As used herein, "cellular sample" and "cell sample" refer to any sample containing intact cells, such as cell cultures, bodily fluid samples and surgical specimens taken for pathological, histological, or cytological interpretation. Suitable samples include, for example, body fluid sample (such as whole blood, bone marrow, urine, semen, saliva, sputum, nipple discharge, breast milk, 5 synovial fluid, cerebrospinal fluid (CSF), ascites fluid, peritoneal fluid, pericardial fluid, bile, gastric fluid, mucus, lymphatic fluid, perspiration, lacrimal fluid, vomit, pleural fluid, cerumen, nasal discharge/secretions, or skene's gland fluid), body fluid fractions (such as blood fractions, including plasma, buffy coat , and erythrocyte fractions), fine needle aspirates (such as bone marrow aspirate), washings (such as bronchial lavage, bronchoalveolar lavage, nasal lavage, douche, or enema), and scrape or brush samples (such as scrapings or brushes from the cervix, anus, mouth, esophagus, stomach, or bronchi).

As used herein, a "detectable moiety" refers to a molecule or material that can produce a detectable signal (such as visually, electronically or otherwise) that indicates the presence (i.e. qualitative analysis) and/or concentration (i.e. quantitative analysis) of the detectable moiety deposited on a sample. A detectable signal can be generated by any known or yet to be discovered mechanism including absorption, emission and/or scattering of a photon (including radio frequency, microwave frequency, infrared frequency, visible frequency and ultra-violet frequency photons). A "detectable moiety" may include chromogenic, fluorescent, phosphorescent, and luminescent molecules and materials, catalysts (such as enzymes) that convert one substance into another substance to provide a detectable difference (such as by converting a colorless substance into a colored substance or vice versa, or by producing a precipitate or increasing sample turbidity). In particular, according to the invention, the detectable moiety is a fluorophore, which belongs to several common chemical classes including coumarins, fluoresceins (or fluorescein derivatives and analogs), rhodamines, resorufins, luminophores and cyanines. Additional examples of fluorescent molecules can be found in Molecular Probes Handbook - A Guide to Fluorescent Probes and Labeling Technologies, Molecular Probes, Eugene, OR, ThermoFisher Scientific, 11th Edition. In other embodiments, the detectable moiety is a molecule detectable via brightfield microscopy, such as dyes including diaminobenzidine (DAB), 4-(dimethylamino) azobenzene-4'-sulfonamide (DABSYL), tetramethylrhodamine (DISCOVERY Purple), N,N'-biscarboxypentyl-5,5'-disulfonato-indo-dicarbocyanine (Cy5), and Rhodamine 110 (Rhodamine). In other examples not being part of the claimed invention, the detectable moiety is a nanoparticle, such as a gold or silver nanoparticle. Other detectable moieties exist or may be developed in the future and should be considered within the scope of "detectable moiety."

As known to one skilled in the art, a Romanowsky-type stain is metachromatic stain useful for staining cytology samples, wherein the stain includes a cationic thiazine dye (such as polychrome methylene blue, azure A, azure B, azure C, azure IV, symdimethylthionine, thionine, methylene violet Bernsthen, methylthionoline, toluidine blue, and combinations thereof) and an anionic halogenated fluorescein dye (such as eosin A, eosin Y, eosin G, and combinations thereof). Suitable Romanowsky-type stains include, for example, Romanowsky stain, Malachowski stain, Giemsa stain, May-Gruenwald stain, May-Gruenwalkd-Giemsa (MGG) stain, Jenner stain, Wright stain, Leishman stain, and DIFF-QUICK (proprietary modified Wright stain). For Romanowsky-type staining, a sample can be fixed in a fixative. Suitable fixatives include, for example, alcohol-based fixatives (e.g., methanol) and aldehyde-based fixatives (e.g., formaldehyde such as buffered formalin).

As used herein, "detection reagent" refers to any reagent that is used to deposit a stain in proximity to a biomarker-specific reagent bound to a cellular sample. Suitable detection reagents include, for example, primary detection reagents (such as a detectable moiety directly conjugated to an antibody), secondary detection reagents (such as secondary antibodies capable of binding to a primary antibody), tertiary detection reagents (such as tertiary antibodies capable of binding to secondary antibodies), enzymes directly or indirectly associated with the biomarker-specific reagent, chemicals reactive with such enzymes to effect deposition of a fluorescent or chromogenic stain, wash reagents used between staining steps, and the like.

As used herein, "specific detection reagent" refers to any composition of matter that is capable of specifically binding to a target chemical structure in the context of a cellular sample. As used herein, the phrase "specific binding," "specifically binds to," or "specific for" or other similar iterations refers to measurable and reproducible interactions between a target and a specific detection reagent, which is determinative of the presence of the target in the presence of a heterogeneous population of molecules including biological molecules. For example, an antibody that specifically binds to a target is an antibody that binds this target with greater affinity, avidity, more readily, and/or with greater duration than it binds to other 10 targets. In one embodiment, the extent of binding of a specific detection reagent to an unrelated target is less than about 10% of the binding of the antibody to the target as measured, e.g., by a radioimmunoassay (RIA). In certain embodiments, a biomarker-specific reagent that specifically binds to a target has a dissociation constant (Kd) of ≤1 µM, ≤100 nM, ≤10 nM, ≤1 nM, or ≤0.1 nM. In another embodiment, specific binding can include, but does not require exclusive binding. Exemplary specific detection reagents include nucleic acid probes specific for particular nucleotide sequences; antibodies and antigen binding fragments thereof; and engineered specific binding compositions, including ADNECTINs (scaffold based on 10th FN3 fibronectin; Bristol-Myers-Squibb Co.), AFFIBODYs (scaffold based on Z domain of protein A from S. aureus; Affibody AB, Solna, Sweden), A VIMERs (scaffold based on domain A/LDL receptor; Amgen, Thousand Oaks, CA), dAbs (scaffold based on VH or VL antibody domain; GlaxoSmithKline PLC, Cambridge, UK), DARPins (scaffold based on Ankyrin repeat proteins; Molecular Partners AG, Zürich, CH), ANTICALINs (scaffold based on lipocalins; Pieris AG, Freising, DE), NANOBODYs (scaffold based on VHH (camelid Ig); Ablynx N/V, Ghent, BE), TRANS-BODYs (scaffold based on Transferrin; Pfizer Inc., New York, NY), SMIPs (Emergent Biosolutions, Inc., Rockville, MD), and TETRANECTINs (scaffold based on C-type lectin domain (CTLD), tetranectin; Borean Pharma A/S, Aarhus, DK). Descriptions of such engineered specific binding structures are reviewed by Wurch et al., Development of Novel Protein Scaffolds as Alternatives to Whole Antibodies for Imaging and Therapy: Status on Discovery Research and Clinical Validation, Current Pharmaceutical Biotechnology, Vol. 9, pp. 502-509 (2008).

As known to one of ordinary skill in the art, a fluorescence label is a detectable moiety that is suitable for staining biomarkers for fluorescence microscopy. Examples include fluorescent and phosphorescent dyes and nanomaterials (such as quantum dots).

As used herein, the term "biomarker" shall refer to any molecule or group of molecules found in a biological sample that can be used to characterize the biological sample or a subject from which the biological sample is obtained. For example, a biomarker may be a molecule or group of molecules whose presence, absence, or relative abundance is: characteristic of a particular cell or tissue type or state; and/or characteristic of a particular pathological condition or state; and/or indicative of the severity of a pathological condition, the likelihood of progression or regression of the pathological condition, and/or the likelihood that the pathological condition will respond to a particular treatment. As another example, the biomarker may be a cell type or a microorganism (such as a bacterium, mycobacterium, fungus, virus, and the like), or a substituent molecule or group of molecules thereof.

As used herein, a "biomarker-specific reagent" refers to a specific detection reagent that is capable of specifically binding directly to a biomarker in the cellular sample. Examples include a primary antibodies immunoreactive with biomarkers of the sample and nucleic acid hybridization probes complementary to nucleic acid biomarkers of the sample.

As used herein, a "brightfield label" refers to a detectable moiety that is suitable for staining cellular samples for brightfield microscopy. Examples include chromogenic dyes, metallographic dyes, and chromophore-containing dyes capable of being converted from a species that does not adhere to a cellular sample to a species that is capable of adhering to the cellular sample (such as DAB).

As used herein, "direct assay" refers to a process involving staining a biomarker in a cellular sample by binding a biomarker-specific reagent conjugated directly with a detectable moiety to biomarkers within the sample in a manner that regions of the sample containing biomarker may be detected microscopically by observing the detectable moiety. Examples include immunohistochemistry (IHC), immunocytochemistry (ICC), chromogenic in situ hybridization (CISH), fluorescent in situ hybridization (FISH), and silver in situ hybridization (SISH) with directly labeled conjugates. Advantages of direct assays include reduction in amount of reagents used and therefore costs, reduction of time to completion of assay, and an ability to detect the biomarkers and Romanowsky or other stains on the same cells.

As used herein, "affinity assay" refers to a process involving staining a biomarker in a cellular sample by binding a biomarker-specific reagent to biomarkers within the sample in a manner that deposits a detectable moiety on the sample in proximity to the biomarker-specific reagent bound thereto, such that regions of the sample containing biomarker may be detected microscopically. Examples include immunohistochemistry (IHC), immunocytochemistry (ICC), chromogenic in situ hybridization (CISH), fluorescent in situ hybridization (FISH), and silver in situ hybridization (SISH).

As used herein, "immunoenzymatic assay" refers to an affinity enzymatic assay in which the biomarker-specific reagent is an antibody.

As used herein, "multiplex stain" refers to an affinity assay in which multiple biomarker-specific reagents that bind to different biomarkers are applied to a single cell sample and stained with different color stains.

As used herein, "affinity enzymatic reaction" refers to an affinity assay in which the biomarker specific reagent localizes an enzyme (such as a peroxidase enzyme or a phosphatase enzyme) to regions of the sample that contain the biomarker, and a set of detection reagents is reacted with the enzyme to deposit a dye on the sample.

As used herein, "antibody" is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

As used herein, "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')2; diabodies; linear antibodies; single-chain antibody molecules (e.g. scFv); and multispecific antibodies formed from antibody fragments.

The term "monoclonal antibody" is used according to its ordinary meaning as understood by one skilled in the art to refer to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, or a combination thereof.

As used herein, "secondary detection reagent" refers to a specific detection reagent capable of specifically binding to a biomarker-specific reagent.

When used as a noun, the term "stain" refers to any substance that can be used to visualize specific molecules or structures in a cellular sample for microscopic analysis, including brightfield microscopy, fluorescent microscopy, electron microscopy, and the like. When used as a verb, the term "stain" refers to any process that results in deposition of a stain on a cellular sample.

Generally, solid support can be implemented as any of a wide variety of different sample carriers. Sample carriers can be planar (e.g., microscope slides, coverslips, plates, trays, and other members that extend in two dimensions and have a relatively narrow thickness). Alternatively, sample carriers can be non-planar, and can be implemented as cups, tubes, vials, and other similar containers, with cross-sectional shapes that include, but are not limited to, circular, elliptical, square, rectangular, triangular, and other polygonal shapes. The type of sample carrier used can depend on the type of sample and process requirements in a preparative workflow. For example, to support tissue samples, planar microscope slides and coverslips can be used. Where the sample includes a relatively high proportion of liquid, sample carriers with one or more wells or cups (e.g., a single-well or multi-well sample plate) may be more convenient.

In an embodiment, the solid support is compatible with microscopic evaluation. In an embodiment, the solid support is compatible with brightfield or fluorescence microscopy and allows a substantial portion of cells of interest to remain adhered to the solid support throughout the staining processes described herein. In an embodiment, the solid support is a microscope slide.

Direct staining can be performed by mixing the sample directly with the biomarker-specific reagent or reagents with directly conjugated labels. After an incubation period the sample can be directly placed onto a solid support for further analyses and staining. The sample can be unmodified (e.g. whole blood or a body fluid), or could be pre-processed (e.g., red blood cells lysed from a whole blood preparation). The dispensing of sample and reagents can be done manually or can be performed using an automated platform.

Affinity staining and Romanowsky staining can be performed using an automated advanced staining platform. Automated advanced staining platforms typically include at least: reservoirs of the various reagents used in the staining protocols, a reagent dispense unit in fluid communication with the reservoirs for dispensing reagent to a solid support, a waste removal system for removing used reagents and other waste from the solid support, and a control system that coordinates the actions of the reagent dispense unit and waste removal system. In addition to performing staining steps, many automated advanced staining platforms can also perform steps ancillary to staining (or are compatible with separate systems that perform such ancillary steps), including: slide baking (for adhering the sample to the slide), dewaxing (also referred to as deparaffinization), antigen retrieval, counterstaining, dehydration and clearing, and coverslipping. Examples of automated advanced staining platforms and their various features are the intelliPATH (Biocare Medical), WAVE (Celerus Diagnostics), DAKO OMNIS and DAKO AUTOSTAINER LINK 48 (Agilent Technologies), BENCHMARK (Ventana Medical Systems, Inc.), Leica BOND, and Lab Vision Autostainer (Thermo Scientific) automated slide stainers. Additionally, a number of United States patents disclosing systems and methods for performing automated analyses include U.S. Pat. Nos. 5,650,327, 5,654,200, 6,296,809, 6,352,861, 6,827,901 and 6,943,029, and U.S. Published Patent Application Nos. 20030211630 and 20040052685. Commercially-available staining units typically operate on one of the following principles: (1) open individual slide staining, in which slides are positioned horizontally and reagents are dispensed as a puddle on the surface of the slide containing a tissue sample (such as implemented on the DAKO AUTOSTAINER Link 48 (Agilent Technologies) and intelliPATH (Biocare Medical) stainers); (2) liquid overlay technology, in which reagents are either covered with or dispensed through an inert fluid layer deposited over the sample (such as implemented on VENTANA BenchMark and DISCOVERY stainers); (3) capillary gap staining, in which the slide surface is placed in proximity to another surface (which may be another slide or a coverplate) to create a narrow gap, through which capillary forces draw up and keep liquid reagents in contact with the samples (such as the staining principles used by DAKO TECHMATE, Leica BOND, and DAKO OMNIS stainers). Some iterations of capillary gap staining do not mix the fluids in the gap (such as on the DAKO TECHMATE and the Leica BOND). In variations of capillary gap staining termed dynamic gap staining, capillary forces are used to apply sample to the slide, and then the parallel surfaces are translated relative to one another to agitate the reagents during incubation to effect reagent mixing (such as the staining principles implemented on DAKO OMNIS slide stainers (Agilent)). In translating gap staining, a translatable head is positioned over the slide. A lower surface of the head is spaced apart from the slide by a first gap sufficiently small to allow a meniscus of liquid to form from liquid on the slide during translation of the slide. A mixing extension having a lateral dimension less than the width of a slide extends from the lower surface of the translatable head to define a second gap smaller than the first gap between the mixing extension and the slide. During translation of the head, the lateral dimension of the mixing extension is sufficient to 5 generate lateral movement in the liquid on the slide in a direction generally extending from the second gap to the first gap. *See* WO 2011-139978 A1. It has recently been proposed to use inkjet technology to deposit reagents on slides. *See* WO 2016-170008 A1. This list of staining technologies is not intended to be comprehensive, and any fully or semi-automated system for performing biomarker staining via affinity staining.

In multiplex methods, the biomarker-specific reagents and detection reagents are applied in a manner that allows the different biomarkers to be differentially labeled. One way to accomplish differential labelling of different biomarkers is to select combinations of biomarker-specific reagents and detection reagents that will not result in cross-reactivity between different biomarker-specific reagents or detection reagents (termed "combination staining"). For example, where primary detection reagents are used, each biomarker-specific reagent has a unique detectable moiety that is spectrally differentiable upon detection. Cross-reactivity between biomarker-specific reagents can also be minimized, for example, by selecting primary antibodies that are derived from different animal species (such as mouse, rabbit, rat, and goat antibodies).

In some embodiments, the multiplex method is a fluorescent multiplex method. In some embodiments, the multiplex method is a brightfield multiplex method. In some embodiments, the multiplex method is a nanoparticles detection method. Combinations of multiplex methods can also be used.

For staining of the sample with biomarker-specific reagents and a set of detection reagents, resulting in a detectable moiety on the sample in proximity to biomarkers contained within the sample.

In some embodiments, the detectable moiety is directly conjugated to the biomarker-specific reagent, and thus is deposited on the sample upon binding of the biomarker-specific reagent to its target (generally referred to as a direct labeling method). Direct labeling methods are often more directly quantifiable, but may have lower detection sensitivity than secondary labeling.

To obtain a thin layer of the cell sample, the cell sample is applied to the solid support in a manner that obtains cytology preparation. In an embodiment, the cytology preparation is a thin layer cytology preparation. Exemplary methods of obtaining thin layer cytology preparations from cellular samples include cytocentrifugation, filter transfer, gravity sedimentation, and cell printing. In cytocentrifugation, a cell sample is provided as a liquid sample (such as a suspension in a carrier solution or as a body fluid sample), placed in contact with the solid support, and centrifuged. Force generated by the centrifugation causes the cells to sediment on the surface of the solid support, thereby forming the cytology preparation. The quality and content of the thin layer obtained by cytocentrifugation may be optimized by, for example, manipulating the sample prior to centrifugation, for example, by adjusting cell concentration, liquifying or diluting viscous samples, removing precipitates or debris, lysing erythrocytes in blood samples, fixing the sample, etc. *See generally* Stokes. Typical cytocentrifugation systems include a centrifugation chamber assembly and a rotor. The centrifugation chamber assembly typically includes a solid support and a vessel for carrying the suspension of the cell sample. When assembled, the vessel places a surface of the suspension in contact with a surface of the solid support. Centrifugation chambers can generally be divided into two classes: chambers that facilitate removal of fluid during sedimentation (for example, by placing an absorbent material adjacent to an interface between the vessel and the solid support) and chambers that facilitate retention of the liquid throughout centrifugation (for example, by placing a seal around the periphery of an interface between the vessel and the surface of the solid support). Illustrations of such arrangements can be seen at Stokes at F1. In operation, an assembled centrifugation chamber is attached to the rotor in an orientation such that rotation of the rotor causes the cells of the cell sample to be sediment on the surface of the solid support. Exemplary commercially available cytocentrifugation systems include CYTOSPIN systems from Thermo Scientific. Exemplary protocols for performing cytocentrifugation can be found at, for example, Koh. In some specific embodiments, the sample is a prepared by a cytocentrifugation onto a microscope slide.

In cell printing methods, small volumes (for example, from 0.1 to 10 µl) of a liquid cell sample are deposited at discrete locations on a surface of the solid support, and the deposited sample is allowed to dry on the surface to obtain the cytology preparation. For example, liquid sample may be flowed through an applicator tip that is moved relative to the surface of the solid support (e.g. in parallel rows or in concentric circles on the surface of the solid support), thereby forming a monolayer having a substantially uniform distribution of cells on the surface of the solid support. Exemplary systems for performing cell printing typically include at least an applicator tip for dispensing a known volume of the liquid cellular sample and means for changing the position of the applicator tip relative to the surface of the solid support (e.g. means for moving the tip, means for moving the solid support, or both). Exemplary commercially available cell printing systems include COBAS m 511 integrated hematology analyzer from Roche, various aspects of which are described at US Patent Nos. 8,815,537, 25 9,116,087, 9,217,695, and 9,602,777. Exemplary methodologies for using cell printing systems for generating cytology slides can be found at Bruegel. In some specific embodiments, the sample is a body fluid sample printed on a slide. In some specific embodiments, the sample is a whole blood sample printed on a slide. In a cell printing system such as the COBAS m 511 system, a sample featuring a suspension of cells in a fluid medium is prepared on a sample carrier such as a microscope slide for analysis. Where the sample corresponds to a whole blood sample or a suspension of blood components in a fluid, the cell printing system prepares a layer of cells on the sample carrier. In certain embodiments, the layer of cells that is deposited effectively corresponds to a monolayer in which the cells are approximately homogeneously distributed. The cell layer can include any one or more of red blood cells, white blood cells, and platelets. To deposit the sample on the sample carrier, the system may optionally dilute the sample (e.g., with a buffer solution, a stain solution, or more generally, any diluent material) and an aliquot of the diluted sample is applied to the sample carrier. Following application of the sample, cells within the sample begin to settle to the surface of the sample carrier. If applied under certain conditions, the settled cells do not overlap, and instead form the desired monolayer. In general, cell printing systems such as the COBAS m 511 integrated hematology analyzer include an applicator and a stage that supports the sample carrier. The sample is discharged from the applicator as relative motion occurs between the applicator and stage. By carefully controlling the relative positions of the applicator and stage (as well as various other system parameters), the sample can be applied to the sample carrier in a reproducible manner.

Table 1 provides exemplary protocols for performing methods not being part of the claimed invention, wherein the biomarker staining is performed on a slide.

**Table 1. Protocols for CD markers on a slide**

| **Protocol 1a** | | **Comment:** |
|---|---|---|
| Step 1 | Print | |
| Step 2 | Fix: m511 fixative | |
| Step 3 | Wash/Block | PBS-azide-BSA |
| Step 4 | 1° Ab - add and incubate | extracellular markers only, multiplex gradually if labelled 1°Ab can be used |
| Step 5 | Wash | PBS |
| Step 6* | 2° Ab - add and incubate | * skip step if labelled 1°Ab can be used |
| Step 7* | Wash | PBS, * skip step if labelled 1°Ab can be used |
| | repeat steps 4-7 for multiplexing | Evaluate single CD markers first, increase gradually for multiplexing |
| Step 8 | Detect | |
| Step 9 | Romanowsky stain | |
| Step 10 | Morphology evaluation | |

| **Protocol 1b** | | **Comment:** |
|---|---|---|
| Step 1 | Print | |
| Step 2 | Fix: m511 fixative | |
| Step 3 | Wash/Block | PBS-azide-BSA |
| Step 4 | 1° Ab - add and incubate | intracellular markers only, multiplex gradually if labelled 1°Ab can be used |
| Step 5 | Wash | PBS |
| Step 6* | 2° Ab - add and incubate | * skip step if labelled 1°Ab can be used |
| Step 7* | Wash | PBS, * skip step if labelled 1°Ab can be used |
| | repeat steps 4-7 for multiplexing | Evaluate single CD markers first, increase gradually for multiplexing |
| Step 8 | Detect | |
| Step 9 | Romanowsky stain | |
| Step 10 | Morphology evaluation | |

| **Protocol 1c** | | **Comment:** |
|---|---|---|
| Step 1 | Print | |
| Step 2 | Fix: m511 fixative | |
| Step 3 | Wash/Block | PBS-azide-BSA |
| Step 4 | 1° Ab - add and incubate | include extra- and intracellular markers, multiplex gradually if labelled 1°Ab can be used |
| Step 5 | Wash | PBS |
| Step 6* | 2° Ab - add and incubate | * skip step if labelled 1°Ab can be used |
| Step 7* | Wash | PBS, * skip step if labelled 1°Ab can be used |
| | repeat steps 4-7 for multiplexing | Evaluate single CD markers first, increase gradually for multiplexing |
| Step 8 | Detect | |
| Step 9 | Romanowsky stain | |
| Step 10 | Morphology evaluation | |

| **Protocol 2** | | **Comment:** |
|---|---|---|
| Step 1 | Print | |
| Step 2 | Fix and Romanowsky stain | m511 protocol |
| Step 3 | Morphology evaluation | |
| Step 4 | Wash/Block | PBS-azide-BSA |
| Step 5 | 1° Ab - add and incubate | include extra- and intracellular markers depending on the results of protocol 1c, multiplex gradually if labelled 1°Ab can be used |
| Step 6 | Wash | PBS |
| Step 7* | 2° Ab - add and incubate | * skip step if labelled 1°Ab can be used |
| Step 8* | Wash | PBS, * skip step if labelled 1°Ab can be used |
| | repeat steps 5-8 for multiplexing | Evaluate single CD markers first, increase gradually |
| Step 9 | Detect | |

| **Protocol 3** | | **Comment:** |
|---|---|---|
| Step 1 | Print | |
| Step 2 | Fix and Romanowsky stain | m511 protocol |
| Step 3 | Morphology evaluation | |
| Step 4 | Destain | 95% methanol |
| Step 5 | Wash/Block | PBS-azide-BSA |
| Step 6 | 1° Ab - add and incubate | include extra- and intracellular markers depending on the results of protocol 1c, multiplex gradually if labelled 1°Ab can be used |
| Step 7 | Wash | PBS |
| Step 8* | 2° Ab - add and incubate | * skip step if labelled 1°Ab can be used |
| Step 9* | Wash | PBS, * skip step if labelled 1°Ab can be used |
| | repeat steps 6-9 for multiplexing | Evaluate single CD markers first, increase gradually |
| Step 10 | Detect | |

| **Protocol 4a** | | **Comment:** |
|---|---|---|
| Step 1 | Print | |
| Step 2 | Fix: m511 fixative | m511 protocol |
| Step 3 | Wash | PBS |
| Step 4 | Antigen retreival | 10 min. at 95°C in 95°C preheated antigen retrieval buffer (100 mM Tris, 5% [w/v] urea, pH 9.5) |
| Step 5 | Wash | PBS |
| Step 6 | Block | PBS-azide-BSA |
| Step 7 | 1° Ab - add and incubate | include extra- and intracellular markers depending on the results of protocol 1c, multiplex gradually if labelled 1°Ab can be used |
| Step 8 | Wash | PBS |
| Step 9* | 2° Ab - add and incubate | * skip step if labelled 1°Ab can be used |
| Step 10* | Wash | PBS, * skip step if labelled 1°Ab can be used |
| | repeat steps 7-10 for multiplexing | Evaluate single CD markers first, increase gradually |
| Step 11 | Detect | |

| **Protocol 4b** | | **Comment:** |
|---|---|---|
| Step 1 | Print | |
| Step 2 | Fix: m511 fixative | m511 protocol |
| Step 3 | Permabilization | Incubate the slides for 10 min with PBS containing either 0.1-0.25% Triton X-100 (or 100 µM digitonin or 0.5% saponin). Triton X-100 is not appropriate for membrane-associated antigens since it destroys membranes. |
| Step 4 | Wash | PBS |
| Step 5 | Block | PBS-azide-BSA |
| Step 6 | 1° Ab - add and incubate | intracellular markers only, multiplex gradually if labelled 1°Ab can be used |
| Step 7 | Wash | PBS |
| Step 8* | 2° Ab - add and incubate | * skip step if labelled 1°Ab can be used |
| Step 9* | Wash | PBS, * skip step if labelled 1°Ab can be used |
| | repeat steps 4-7 for multiplexing | Evaluate single CD markers first, increase gradually |
| Step 10 | Detect | |
| Step 11 | Romanowsky stain | |
| Step 12 | Morphology evaluation | |

Table 2 provides exemplary protocols for performing the methods of the present disclosure wherein the biomarker staining is performed in a tube.

**Table 2. CD markers staining in the tube**

| **Protocol 1a** | extracellular markers | **Comment:** |
|---|---|---|
| Step 1 | Add CD marker(s) into the blood sample (extracellular) | Evaluate single CD markers first, increase gradually for multiplexing |
| Step 2 | Add buffer/Block | Buffer used in Boston: BSA blocking buffer, 3% in PBS, with 0.02% sodium azide |
| Step 3 | Incubate | 15 min. at RT in the darkness |
| Step 4 | Print | |
| Step 5 | Detect | Printed sample will be compared to sample measured on flow cytometer |
| Step 6 | Fix and Romanowsky stain | |
| Step 7 | Morphology evaluation | |

| **Protocol 1b** | intracellular markers | **Comment:** |
|---|---|---|
| Step 1 | Fix and permeabilize cells: Add fixing agent into the blood sample | fixing agent (commercially available) |
| Step 2 | Incubate | 15 min. at RT |
| Step 3 | Wash | PBS-azide-BSA (PBS pH 7.3, 0.02% sodium azide, 0.02% BSA, 0.01% EDTA) |
| Step 4 | Add CD marker(s) into the sample (intracellular) | Evaluate single CD markers first, increase gradually for multiplexing |
| Step 5 | Add buffer/Block | Buffer used in Boston: BSA blocking buffer, 3% in PBS, with 0.02% sodium azide |
| Step 6 | Incubate | 15 min. at RT in the darkness |
| Step 7 | Print | |
| Step 8 | Detect | Printed sample will be compared to sample measured on flow cytometer |
| Step 9 | Fix and Romanowsky stain | |
| Step 10 | Morphology evaluation | |

| **Protocol 1c** | extra- and intracellular markers | **Comment:** |
|---|---|---|
| Step 1 | Add CD marker(s) into the blood sample (extracellular) | Evaluate single CD markers first, increase gradually for multiplexing |
| Step 2 | Incubate | 15 min. at RT in the darkness |
| Step 3 | Fix and permeabilize cells: Add fixing agent into the blood sample | fixing agent (commercially available) |
| Step 4 | Incubate | 15 min. at RT |
| Step 5 | Wash | PBS-azide-BSA (PBS pH 7.3, 0.02% sodium azide, 0.02% BSA, 0.01% EDTA) |
| Step 6 | Add CD marker(s) into the sample (intracellular) | Evaluate single CD markers first, increase gradually for multiplexing |
| Step 7 | Add buffer/Block | Buffer used in Boston: BSA blocking buffer, 3% in PBS, with 0.02% sodium azide |
| Step 8 | Incubate | 15 min. at RT in the darkness |
| Step 9 | Print | |
| Step 10 | Detect | Printed sample will be compared to sample measured on flow cytometer |
| Step 11 | Fix and Romanowsky stain | |
| Step 12 | Morphology evaluation | |

| **Protocol 2** | | **Comment:** |
|---|---|---|
| Step 1 | Add CD marker(s) into the blood sample | Evaluate single CD markers first, increase gradually for multiplexing |
| Step 2 | Add buffer/Block | Buffer used in Boston: BSA blocking buffer, 3% in PBS, with 0.02% sodium azide |
| Step 3 | Incubate | 15 min. at RT in the darkness |
| Step 4 | Wash | PBS-azide-BSA (PBS pH 7.3, 0.02% sodium azide, 0.02% BSA, 0.01% EDTA) |
| Step 5 | Print | |
| Step 6 | Detect | Printed sample will be compared to sample measured on flow cytometer |
| Step 7 | Fix and Romanowsky stain | |
| Step 8 | Morphology evaluation | |

| **Protocol 3** | | **Comment:** |
|---|---|---|
| Step 1 | Wash the blood sample | PBS-azide-BSA (PBS pH 7.3, 0.02% sodium azide, 0.02% BSA, 0.01% EDTA) |
| Step 2 | Add CD marker(s) into the blood sample | Evaluate single CD markers first, increase gradually for multiplexing |
| Step 3 | Add buffer/Block | Buffer used in Boston: BSA blocking buffer, 3% in PBS, with 0.02% sodium azide |
| Step 4 | Incubate | 15 min. at RT in the darkness |
| Step 5 | Wash | PBS-azide-BSA (PBS pH 7.3, 0.02% sodium azide, 0.02% BSA, 0.01% EDTA) |
| Step 6 | Print | |
| Step 7 | Detect | Printed sample will be compared to sample measured on flow cytometer |
| Step 8 | Fix and Romanowsky stain | |
| Step 9 | Morphology evaluation | |

| **Protocol 4** | | **Comment:** |
|---|---|---|
| Step 1 | Wash the blood sample | PBS-azide-BSA (PBS pH 7.3, 0.02% sodium azide, 0.02% BSA, 0.01% EDTA) |
| Step 2 | Add CD marker(s) into the blood sample | Evaluate single CD markers first, increase gradually for multiplexing |
| Step 3 | Add buffer/Block | Buffer used in Boston: BSA blocking buffer, 3% in PBS, with 0.02% sodium azide |
| Step 4 | Incubate | 15 min. at RT in the darkness |
| Step 5 | Wash | PBS-azide-BSA (PBS pH 7.3, 0.02% sodium azide, 0.02% BSA, 0.01% EDTA) |
| Step 6 | Lyse RBCs | lysing agent (commercially available) |
| Step 7 | Wash/Resuspend | PBS-azide-BSA (PBS pH 7.3, 0.02% sodium azide, 0.02% BSA, 0.01% EDTA) |
| Step 8 | Print | |
| Step 9 | Detect | Printed sample will be compared to sample measured on flow cytometer |
| Step 10 | Fix and Romanowsky stain | |
| Step 11 | Morphology evaluation | of WBC |

### CD experiments

### Materials

Antibodies at various concentrations (e.g., 2 mg/ml and 200 µg/ml). CD45 from Roche Penzberg at 2 mg/ml concentration. CD20 (Santa Cruz Biotechnology, Inc., Dallas, TX)

Incubation buffer for antibody dilution (3% BSA in PBS containing 0.02% sodium azide).

### Whole blood sample

Protocol 1. Single Antibody with concentration of 2 mg/ml: (e.g. CD45).

1 µl was added to 99 µl Incubation Buffer to create Stock Solution #1 of 20 µg/ml antibody. 5 µl of Stock Solution #1 was mixed with 45 µl of Whole Blood to create a final antibody concentration of approximately 2 µg/ml. The mixture was incubated at room temperature in the dark for 15 minutes and then placed on the Cobas m 511 and a slide was produced for imaging.

Protocol #2. Single Antibody with concentration of 200 µg/ml (e.g. CD20).

20 µl was added to 180 µl Incubation Buffer to create Stock Solution #2 of 20 µg/ml antibody. 5 µl of Stock Solution #2 was mixed with 45 µl of Whole Blood to create a final antibody concentration of approximately 2 µg/ml. The mixture was incubated at room temperature in the dark for 15 minutes and then placed on the COBAS m 511 and a slide was produced for imaging.

Protocol #3: Multiplexed Antibody studies (e.g. CD45 and CD20).

Stock Solution #1 and Stock Solution #2 from Protocols #1 and #2 were used. 5 µl of Stock Solution #1 and 5 µl of Stock Solution #2 were mixed with 40 µl of Whole Blood to achieve a final antibody concentration of approximately 2 µg/ml of each antibody. The mixture was incubated at room temperature in the dark for 15 minutes and then placed on the COBAS m 511 and a slide was produced for imaging.

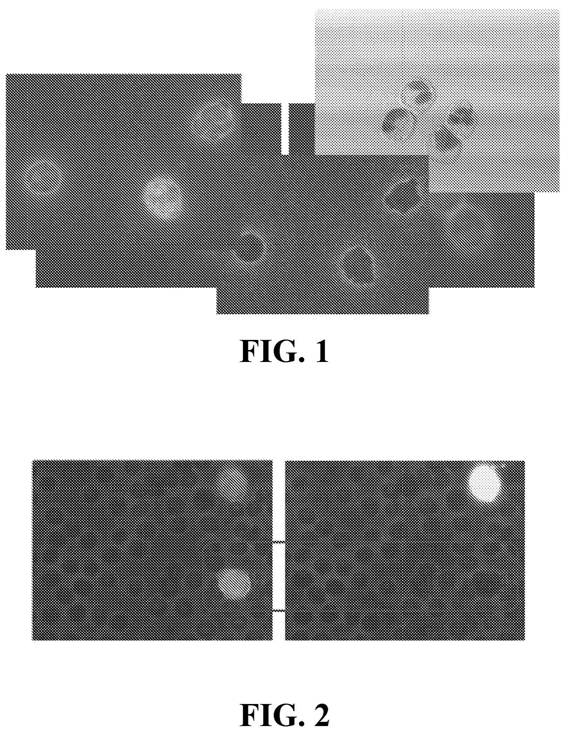

As shown in FIG. 1A, cells were positively stained for CD 45 and Romanowsky-type staining.

As shown in FIG. 1B, the method distinguished between CD 45 positive/CD 20 positive cells and CD 45 positive/CD 20 negative cells on the same slide.

### Example: CD 45 and CD 14-APC

CD 45-PerCP was obtained from Roche Penzberg at 1.26 mg/ml concentration. A CD 45 stock solution used in the concentration of 55.44 µg/ml (4.4 µL added to 95.4 µl of BSA blocking buffer, 3% in PBS, with 0.02% sodium azide), final concentration in the sample 5 µg/ml. CD 14-APC was obtained from Beckman Coulter (REF IM2580) and was used at the recommended concentration of 10 µl/100 µl sample. The sample tested: 100 µl EDTA blood + 10 µl Stock solution CD 45 + 10 µl CD 14. The mixture was incubated for 15 minutes in the dark, at room temperature, afterwards the slide was printed on COBAS m511. After fluorescence detection/imaging slide was Romanowsky stained on COBAS m511 and imaged on brightfield microscope.

Example: CD 45 from Roche Penzberg used us described above - Slide 1. CD 45 from BioLegend Catalog#368506 used: 50 µl EDTA blood + 10 µl CD 45 - Slide 2. For each slide separately: mixture was incubated 15 minutes in the dark, at room temperature, afterwards slide was printed on COBAS m511. After fluorescence detection/imaging slide was Romanowsky stained on COBAS m511 and imaged on brightfield microscope.

Example: CD 45-PerCP obtained from Roche Penzberg at 1.26 mg/mL concentration: Stock solution: concentration of 30 ug/mL (final concentration in the sample 5 µg/ml). CD 19-APC obtained from Roche Penzberg at 0.47 mg/ml concentration: Stock solution: concentration of 120 µg/ml (final concentration in the sample 20 µg/ml). CD 3-AlexaFluor488 obtained from Roche Penzberg at 1.7 mg/ml concentration: Stock solution: concentration of 60 µg/ml (final concentration in the sample 10 µg/ml).

Stock solution containing all 3 CD markers at above concentrations was prepared by adding 2.4 µl of CD 45, 25.5. µl of CD19, 3.5 µl of CD3 and 68.6 µl of PBS buffer: sample tested: 50 µl EDTA blood + 10 µl Stock solution. The mixture was incubated 15 minutes in the dark, at room temperature, afterwards a slide was printed on COBAS m511. After fluorescence detection/imaging slide was Romanowsky stained on COBAS m511 and imaged with brightfield microscopy.

### Example: BD Multitest 6-color TBNK (Catalog No. 644611)

Sample tested: 50 µl EDTA blood + 10 µl BD Multitest 6-color TBN. The mixture was incubated 15 minutes in the dark, at room temperature, afterwards a slide was printed on COBAS m511. After fluorescence detection/imaging slide was Romanowsky stained on COBAS m511 and imaged with brightfield microscopy.

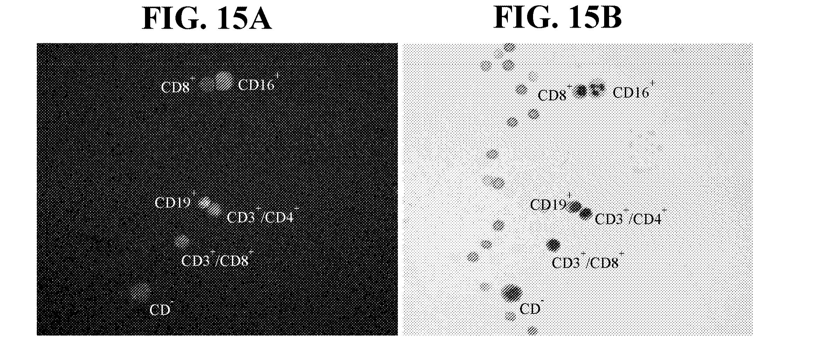

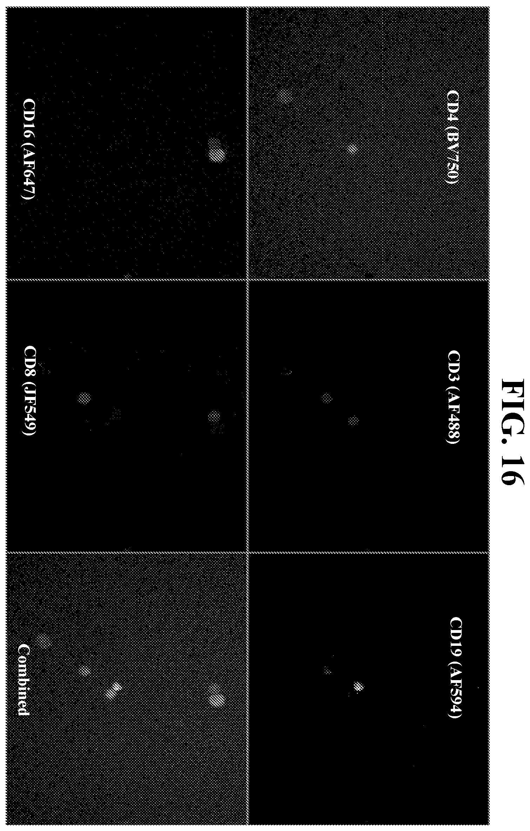

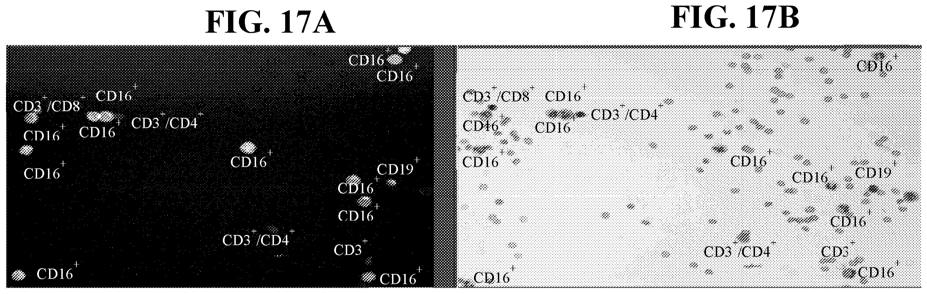

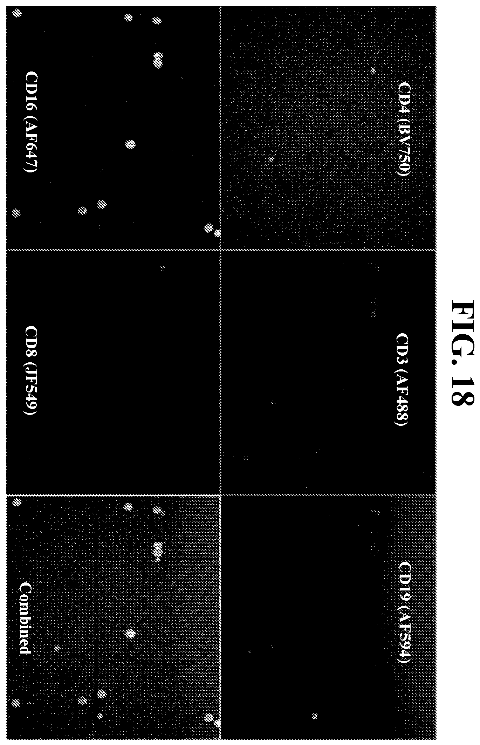

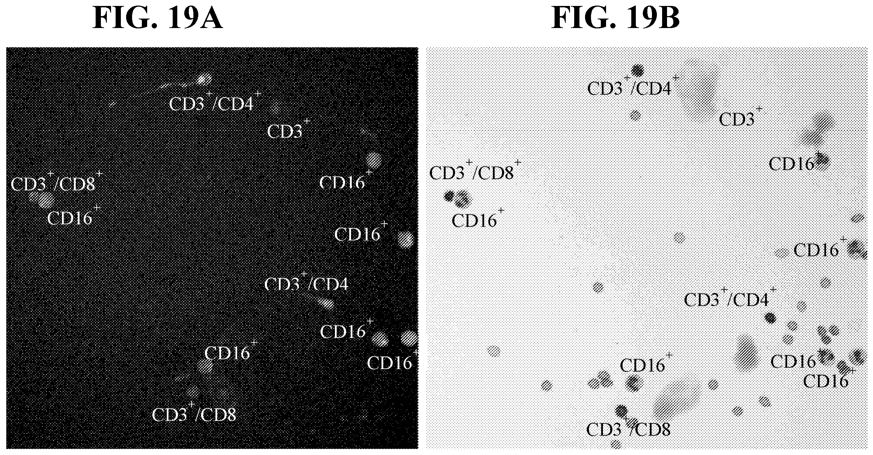

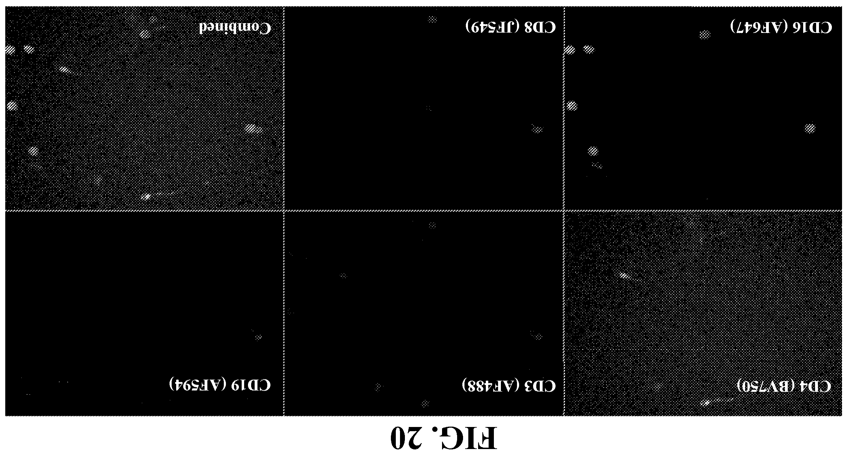

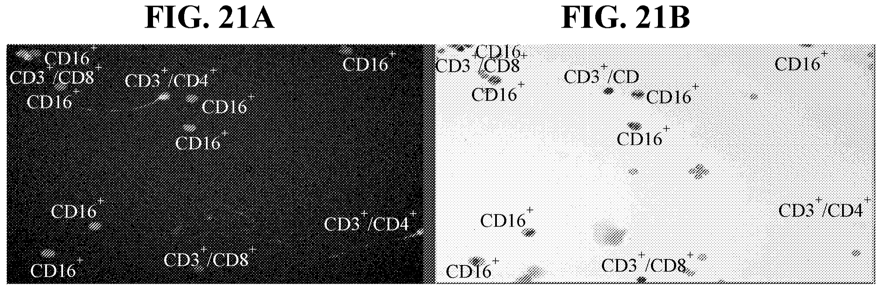

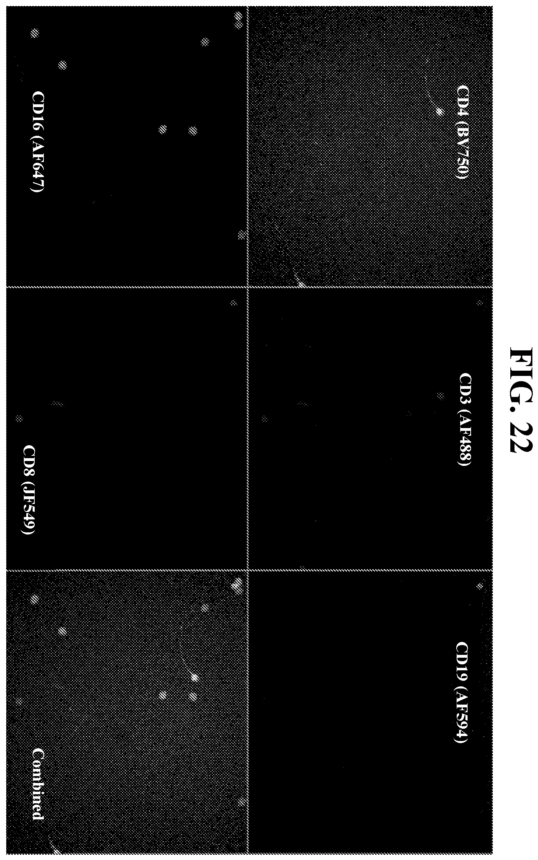

FIGS. 15-22 depict multiplex staining for CD3, CD4, CD8, CD16, and CD19 with corresponding Romanowsky staining imaged with brightfield microscopy.

Following collection of a whole blood sample from a subject, biomarker detection reagents (fluorescently labeled primary antibodies to each specific biomarker) were added to the sample. An aliquot of the sample was then printed on a microscope slide and imaged by fluorescence microscopy. After obtaining fluorescent images of the sample, the sample was fixed and Romanowsky stained on the microscope slide. Cell morphology in the Romanowsky stained sample was then obtained by brightfield microscopy. Fluorescence images were merged and compared with brightfield images.

In other experiments, red blood cells contained in the whole blood sample were lysed. The sample was then washed following the lysis step to remove cellular debris and material contained in the whole blood sample, and to concentrate white blood cells in the sample. The washed cell sample was then stained with biomarker detection reagents, washed to remove any unbound reagents, printed on a microscope slide and imaged for fluorescent staining, followed by preparation for Romanowsky staining and imaging for cell morphology.

The compositions and methods of the present disclosure advantageously allows for a side-by-side comparison of cells stained for one or more biomarkers and Romanowsky-stained to analyze cell morphology. The methods are less complex and reduce costs because of the reduction in the amount and types of reagents used. The methods significantly lower incubation time and use fewer processing steps.

## Claims

1. A method for detecting a biomarker and morphology in a cell sample, the method comprising:
contacting a cell sample with one or more biomarker-specific reagents that specifically binds to a biomarker in the cell sample, wherein the one or more biomarker-specific reagents comprises a fluorescent label;
depositing the biomarker-stained cell sample on a solid support;
analyzing the biomarker-stained cell sample for one or more biomarker;
staining the biomarker-stained cell sample with a Romanowsky-type stain to obtain a Romanowsky-type stained cell sample; and
analyzing the Romanowsky-type stained cell sample to determine morphology of at least one cell in the Romanowsky-type stained cell sample.

2. The method of claim 1, wherein the sample is a body fluid sample.

3. The method of claim 1, wherein the solid support is selected from a microscope slide, a coverslip, a plate, a tray, a cup, a tube, a vial, and combinations thereof.

4. The method of claim 1, wherein the cell sample is deposited on the solid support in a monolayer.

5. The method of claim 1, wherein the cell sample is deposited on the solid support by printing the cell sample on the solid support.

6. The method of claim 1, performed on an automated staining platform.

## Patentansprüche

1. Verfahren zum Nachweis eines Biomarkers und einer Morphologie in einer Zellprobe, wobei das Verfahren Folgendes umfasst:
Inkontaktbringen einer Zellprobe mit einem oder mehreren Biomarker-spezifischen Reagenzien, die spezifisch an einen Biomarker in der Zellprobe binden, wobei das eine oder die mehreren Biomarker-spezifischen Reagenzien eine fluoreszierende Markierung umfassen;
Aufbringen der Biomarker-gefärbten Zellprobe auf einen festen Träger;
Analysieren der Biomarker-gefärbten Zellprobe auf einen oder mehrere Biomarker;
Färben der Biomarker-gefärbten Zellprobe mit einem Romanowsky-Farbstoff, um eine Romanowsky-gefärbte Zellprobe zu erhalten; und
Analysieren der Romanowsky-gefärbten Zellprobe, um die Morphologie mindestens einer Zelle in der Romanowsky-gefärbten Zellprobe zu bestimmen.

2. Verfahren nach Anspruch 1, wobei die Probe eine Körperflüssigkeitsprobe ist.

3. Verfahren nach Anspruch 1, wobei der feste Träger aus einem Objektträger, einem Deckglas, einer Platte, einer Schale, einem Becher, einem Röhrchen, einem Fläschchen und Kombinationen davon ausgewählt ist.

4. Verfahren nach Anspruch 1, wobei die Zellprobe in einer Monoschicht auf den festen Träger aufgebracht wird.

5. Verfahren nach Anspruch 1, wobei die Zellprobe auf den festen Träger durch Drucken der Zellprobe auf den festen Träger aufgebracht wird.

6. Verfahren nach Anspruch 1, das auf einer automatisierten Färbeplattform durchgeführt wird.

## Revendications

1. Procédé de détection d'un biomarqueur et d'une morphologie dans un échantillon de cellule, le procédé comprenant :
la mise en contact d'un échantillon de cellule avec un ou plusieurs réactifs spécifiques à un biomarqueur qui se lient spécifiquement à un biomarqueur dans l'échantillon de cellule, dans lequel le ou les réactifs spécifiques à un biomarqueur comprennent un marqueur fluorescent ;
le dépôt de l'échantillon de cellule à coloration par biomarqueur sur un support solide ;
l'analyse de l'échantillon de cellule à coloration par biomarqueur pour un ou plusieurs biomarqueurs ;
la coloration de l'échantillon de cellule à coloration par biomarqueur avec une coloration de type Romanowsky pour obtenir un échantillon de cellule à coloration de type Romanowsky ; et
l'analyse de l'échantillon de cellule à coloration de type Romanowsky pour déterminer la morphologie d'au moins une cellule dans l'échantillon de cellule à coloration de type Romanowsky.

2. Procédé selon la revendication 1, dans lequel l'échantillon est un échantillon de fluide corporel.

3. Procédé selon la revendication 1, dans lequel le support solide est choisi parmi une lame de microscope, une lamelle, une plaque, un plateau, une coupelle, un tube, un flacon et des combinaisons de ceux-ci.

4. Procédé selon la revendication 1, dans lequel l'échantillon de cellule est déposé sur le support solide en une monocouche.

5. Procédé selon la revendication 1, dans lequel l'échantillon de cellule est déposé sur le support solide par impression de l'échantillon de cellule sur le support solide.

6. Procédé selon la revendication 1, réalisé sur une plateforme de coloration automatisée.
